# EUROPEAN PATENT APPLICATION

(11) **EP 4 821 915 A1**
(43) Date of publication of application: **14.10.2026**
(21) Application number: 25169463.4
(22) Date of filing: 09.04.2025
(51) Int. Cl.: C07C 229/12, A61P 43/00, C07D 201/00, A61K 9/51, C07D 295/15

(54) **NOVEL SYMMETRICAL IONIZABLE LIPIDS BASED ON A GLYCEROL TRIESTER CORE AND THEIR USES IN LIPID NANOPARTICLES**

(71) Applicant: Bayer Aktiengesellschaft, 51373 Leverkusen (DE)
(72) Inventor: The designation of the inventor has not yet been filed
(74) Representative: BIP Patents

(57) **Abstract**

Disclosed herein are ionizable lipids defined as compounds of Formula (I or pharmaceutically acceptable salts thereof. Also disclosed are lipid nanoparticles comprising a compound of Formula or a pharmaceutically acceptable salt thereof; pharmaceutical compositions comprising a plurality of lipid nanoparticles comprising a compound of Formula (I), as well as methods for delivering a nucleic acid segment comprising administering a plurality of lipid nanoparticles comprising a compound of Formula (I) or a pharmaceutically acceptable salt thereof, and a nucleic acid segment.

## Description

Delivery of drug delivery systems poses challenges in fields of chemistry, biology, and medicine. For example, drug delivery systems are hindered due to poor understanding of how molecular properties of a system control delivery to tissues and confer drug efficacy.

The present invention addresses the need for compositions, preparations, nanoparticles, and/or nanomaterials and methods of their use. It recognizes that structural features of these entities impact functional responses in vivo, in vitro, and ex vivo. For example, the selection and combination of components can influence the functional activity of lipid nanoparticles. Functional activity may refer to desired tropisms, stabilization, and/or drug delivery efficacy. Different ratios of components can affect one or more functional activities of compositions, preparations, nanoparticles, and/or nanomaterials.

Additionally, the chemical structures of lipids can confer improved properties compared to reference lipid structures. Compounds of Formula I, 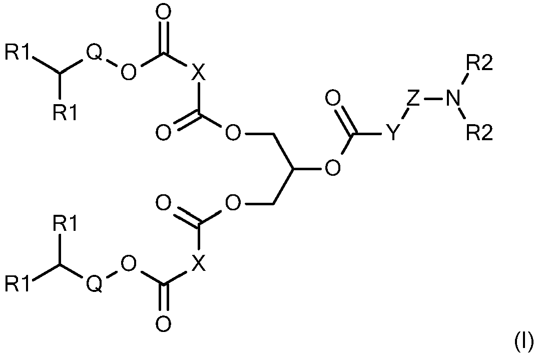 R1, R2, X, Y, Z is defined herein.

Drug delivery systems face challenges in chemistry, biology, and medicine due to limited understanding of molecular properties affecting tissue delivery and drug efficacy. This invention addresses the need for effective compositions and nanoparticles by recognizing that structural features impact responses in vivo, in vitro, and ex vivo. For example, lipid nanoparticle components can influence tropisms, stabilization, and drug delivery efficacy, with different ratios affecting functional activities.

Additionally, the chemical structures of lipids can confer improved properties compared to reference lipid structures. Compounds of Formula I, or a pharmaceutically acceptable salt thereof, are described, where each of R1, R2, X, Y, Z is defined herein.

Among other things, the present disclosure recognizes that lipid nanoparticle (LNP) compositions comprising one or more ionizable lipids. For example, the present disclosure provides that LNP compositions and/or preparations comprising one or more of the disclosed ionizable lipids conferred unexpected tropisms.

In some embodiments, provided compositions, preparations, nanoparticles, and/or nanomaterials are for use in methods of treatment, delivery, producing polypeptides, or delaying/arresting progression of a disease or disorder.

In some embodiments, provided compositions, preparations, nanoparticles, and/or nanomaterials are for use in methods of manufacturing.

The delivery of drug delivery systems presents challenges in the fields of chemistry, biology, and medicine. For example, these systems face obstacles due to limited knowledge of how molecular properties control tissue delivery and affect drug efficacy.

The current invention addresses the need for compositions, preparations, nanoparticles, and methods of their use. It is recognized that structural features of these components impact functional responses in vivo, in vitro, and ex vivo. For instance, the selection and combination of various components can influence the functional activity of lipid nanoparticles. This functional activity may include desired tropisms, stabilization, and drug delivery efficacy. Additionally, different ratios of the components can affect one or more functional activities of the described compositions, preparations, nanoparticles, and nanomaterials.

The present disclosure demonstrates various properties of ionizable lipids, such as tropism, stabilization, and delivery efficacy of cargos like therapeutic or prophylactic agents, which include a trivalent core derived from glyceroll. It also covers compositions, preparations, nanoparticles, and/or nanomaterials (like LNPs and related compositions, preparations, nanoparticles, and/or nanomaterials) and methods of their use.

The disclosure identifies that lipid nanoparticle (LNP) compositions can include one or more ionizable lipids. For instance, it indicates that LNP compositions and preparations containing the disclosed ionizable lipids demonstrate specific tropisms.

In certain embodiments, the provided compositions, preparations, nanoparticles, and nanomaterials are intended for use in methods of treatment, delivery, producing polypeptides, or delaying/arresting the progression of diseases or disorders. Additionally, some embodiments are intended for use in characterization methods.

Elements involving one aspect of the invention, such as methods, can be applied to other aspects and vice versa.

### BACKGROUND

The present invention covers ionizable lipids defined by compounds of general formula (I) which are inter alia useful as ionizable lipids in lipid nanoparticles (LNPs).

### Prior art

The prior art relevant to the present invention includes several references that broadly cover similar chemical compounds but lack specific examples or focus on the particular compounds claimed herein.

In one embodiment the the ionizable lipids according to this invention exhibit a glycerol triester core.

In yet one embodiment the the ionizable lipids according to this invention exhibit a glycerol triester core and a symmetric (achiral) setup of lipophilic side chains i.e. those side chains that do not carry the ionizable tertiary amine.

WO2023/121975 - This document only discloses a glycerol core in a generic way. In a specific or exemplified way it discloses 1,3,5-pentanetriol-, 1,4,7-heptanetriol-, or 1,5,9-nonanetriol-cores. WO2020/219876 discloses with two exceptions only compounds with a homoglycerol backbone. In all of the specifically disclosed compounds the two lipid side chains are not identical, thus resulting in chiral compounds.

JP06279784 discloses structuraly related compounds in a very generic fashion from a different technical field (bleaching activators).

WO2021/005103, discloses structuraly related compounds in a very generic fashion. This disclosure provides neither a focus nor an exemplification towards the ionizable lipids according to this invention.

WO2005/113541, WO2023056917 disclose structuraly related compounds with a different backbone structure.

WO2023/186171 discloses structuraly related compounds in a very generic fashion, which are further centered on nitrogen. All are apparently Michael acceptors (a,b-unstable esters).

WO1999/037621 discloses alpha-amino acid with N-sulfonyl substituents, in which the backbone of the ionizable lipids of this invention are only disclosed as substituents.

In summary, while the prior art mentions compounds that share some structural features with those of the present invention, none of the references provide a clear description or exemplification of the specific name compounds of general formula (I) claimed herein.

In particular, the compounds of the present invention can be used as ionizable lipids or are precursors for the synthesis of such ionizable lipids. One particular aspect of the ionizable lipids of this disclosure is their surprisingly suitability in the formation of Lipid Nanoparticles (LNPs) /that have been found to deliver cargo within the LNP into cells with high efficiency and/or high cell or organ type specificity (tropism) for which data are given in biological experimental section. These LNPs have a wide variety of applications inter alia in the treatment of a wide range of diseases.

### BRIEF DESCRIPTION OF THE FIGURES

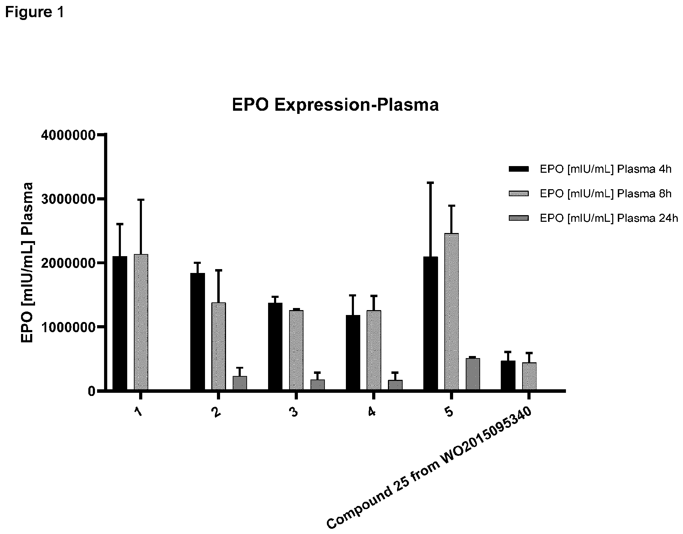

**FIG. 1** provides a graphical representation of the results from the in vivo testing according in mice using EPO as a reporter. Black bars represent EPO values in plasma after 4h, light gray after 8h, and dark grey after 24 h.

### DESCRIPTION OF THE INVENTION

### Ionizable Lipids

In accordance with a first aspect, the present invention covers compounds of general formula (I):

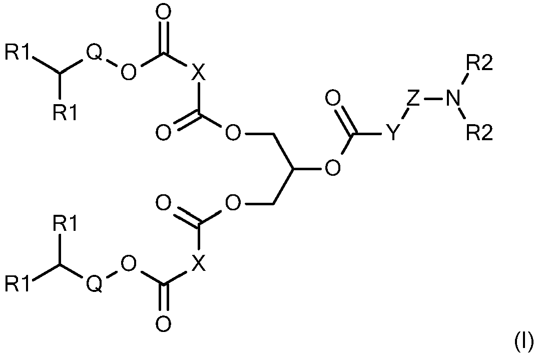

In which
- R1: is C₅-C₁₀ alkyl, optionally substituted with 1 to 3 C₁-C₄ alkyl, and or 1-3 methoxy (OMe) or ethoxy (OEt) groups,
- Q:: direct bond, CH₂, C₂-C₅ alkanediyl,
- X: is C₁-C₄ alkanediyl,
- Y: is a direct bond, oxygen, nitrogen or sulfur,
- Z: is C₂-C₈ alkanediyl, optionally substituted with 1 to 3 C₁-C₄ alkyl, and or 1-3 OMe or OEt groups,
- R2: is C₁-C₃ alkyl, optionally substituted with 1 to 3 C₁-C₃ alkyl, and or 1-2 OMe or OEt groups, both R2 can also - together with the N they are connected to - form a saturated ring with 4 to 8 members, with 0-3 additional O, N or S in the ring and with 1 to 3 C₁-C₃ alkyl, and or 1-2 OMe or OEt groups, and where the additional substituents can form a second ring, and wherein R2-N-R2 stays symmetrical, and wherein the resulting molecule remains achiral.

In certain embodiments
- R1: is linear or branched C₅-C₁₀ alkyl,
- Q:: direct bond, CH₂, C₂-C₅ alkanediyl,
- X: is C₁-C₄ alkanediyl,
- Y: is a direct bond, oxygen, nitrogen or sulfur,
- Z: is linear or branched C₃-C₆ alkanediyl
- R2: both R2 can also - together with the N they are connected to - form a saturated ring with 4 to 8 members, with 0-3 additional O, N or S in the ring and with 1 to 3 C₁-C₃ alkyl, and or 1-2 OMe or OEt groups, and where the additional substituents can form a second ring, and wherein R2-N-R2 stays symmetrical, and wherein the resulting molecule remains achiral.

In certain other embodiments
- R1: is linear or branched C₅-C₁₀ alkyl,
- Q:: direct bond, -CH₂-
- X: is propane-1,3-diyl
- Y: is a direct bond, oxygen, nitrogen or sulfur,
- Z: is linear or branched C₃-C₄ alkanediyl
- R2: both R2 can also - together with the N they are connected to - form a saturated ring with 4 to 8 members, with 0-3 additional O, N or S in the ring and with 1 to 3 C₁-C₃ alkyl, and or 1-2 OMe or OEt groups, and where the additional substituents can form a second ring, and wherein R2-N-R2 stays symmetrical, and wherein the resulting molecule remains achiral.

In particular embodiments
- R1: is octyl,
- Q:: direct bond, -CH₂-
- X: is propane-1,3-diyl
- Y: is a direct bond or oxygen,
- Z: is linear propane-1,3-diyl or butane-1,4-diyl chain,
- R2: is C₁-C₃ alkyl, optionally substituted with 1 to 2 C₁-C₃ alkyl, both R2 can also - together with the N they are connected to - form a saturated ring with 5 to 6 members, optionally 1 additional O, N in the ring and with 1 to 2 C₁-C₃ alkyl or O-Methyl groups, and where the additional substituents can form a second ring and where R2-N-R2 stays symmetrical and thus the whole molecule remains achiral.
and stereoisomers, tautomers, hydrates, solvates, and salts thereof, and mixtures of same.

Exemplary ionizable lipid compounds are listed in Table 1.

| **No:** | **Structure** |
|---|---|
| 1 | 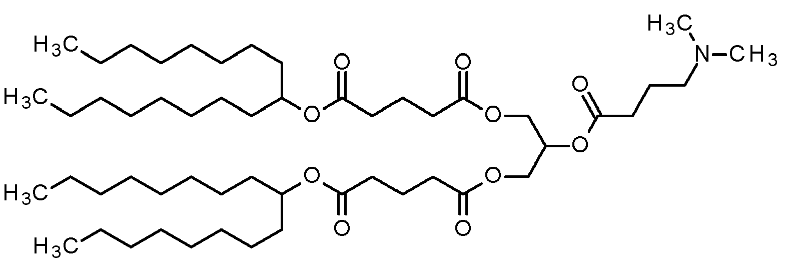 |
| 2 | 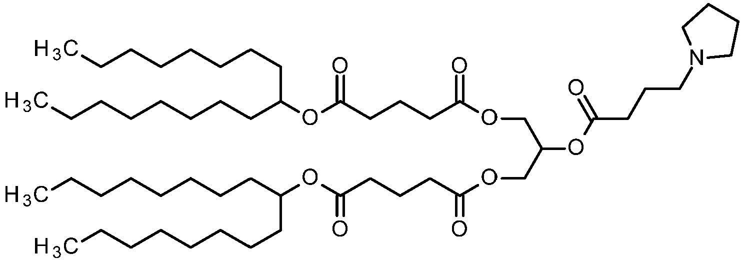 |
| 3 | 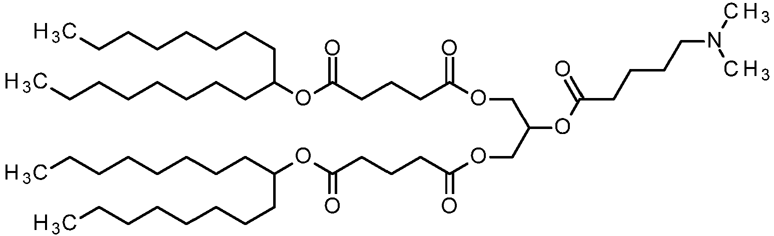 |
| 4 | 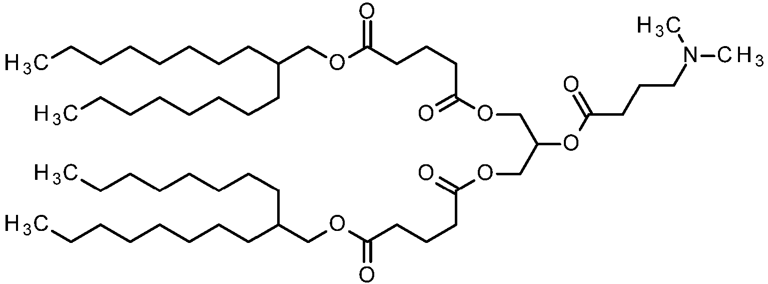 |
| 5 | 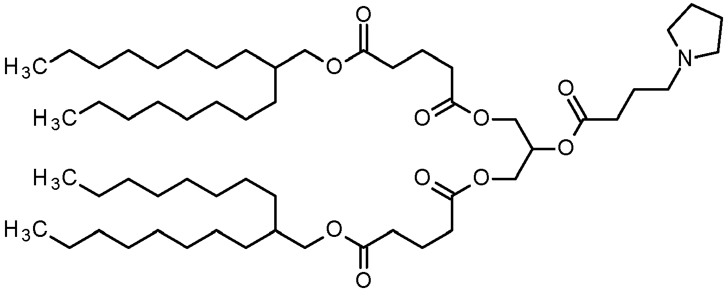 |

### Lipid Nanoparticles (LNPs)

The present invention provides for compositions, preparations, and/or nanomaterials that comprise lipid nanoparticles. In some embodiments, lipid nanoparticles comprise one or more components. In some embodiments, lipid nanoparticles comprise one or more components such as compounds, ionizable lipids, sterols, conjugate-linker lipids, and phospholipids. Among other things, the present disclosure describes that selection and combination of one or more of the components as described herein impacts characteristics of lipid nanoparticles such as diameter, pKa, stabilization, and ionizability.

Among other things, the present disclosure describes that selection and combination of one or more of the components as described herein impacts functional activity of lipid nanoparticles such as tropism, stabilization, and drug delivery efficacy. For example, the present disclosure describes that a combination of components may better suit delivery of siRNA. As another example, the present disclosure describes that a combination of components may better suit delivery of mRNA. As another example, the present disclosure describes that a combination of components may better suit delivery of DNA.

In some embodiments, lipid nanoparticles comprise one or more compounds as described herein. In some embodiments, lipid nanoparticles comprise one or more ionizable lipids as described herein. In some embodiments, lipid nanoparticles comprise one or more sterols as described herein. In some embodiments, lipid nanoparticles comprise one or more conjugate-linker lipids as described herein. In some embodiments, lipid nanoparticles comprise one or more phospholipids as described herein.

In some embodiments, an ionizable lipid is included in a lipid nanoparticle (LNP) preparation at a concentration ranging from approximately 30 mole percent to 70 mole percent, based on the total moles of components of the lipid nanoparticle. In other embodiments, the ionizable lipid concentration ranges from about 33 mole percent to 60 mole percent, from about 34 mole percent to 55 mole percent, or from about 33 mole percent to 51 mole percent. Additionally, specific embodiments include ionizable lipids at concentrations of approximately 34.7 mole percent or 50 mole percent, based on the total moles of components of the lipid nanoparticle.

Among other things, in some embodiments, a lipid nanoparticle composition comprises an ionizable lipid. In some embodiments, a lipid nanoparticle preparation comprises an ionizable lipid; a phospholipid; a conjugate-linker lipid; and a cholesterol. In some embodiments, an ionizable lipid is or comprises a structure according to a compound described herein. In some embodiments, an ionizable lipid is present in a LNP preparation from about 30 mole percent to about 70 mole percent, based on total moles of components of the lipid nanoparticle.

In some embodiments, lipid nanoparticles described herein have an average hydrodynamic diameter from about 30 to about 220 nm. In some embodiments, lipid nanoparticles described herein have an average hydrodynamic diameter that is about 30 nm, 35 nm,40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, 150 nm, 155 nm, 160 nm, 165 nm, 170 nm, 175 nm, 180 nm, 185 nm, 190 nm, 195 nm, 200 nm, 205 nm, 210 nm, 215 nm, 220 nm, or any range having endpoints defined by any two of the aforementioned values. For example, in some embodiments, lipid nanoparticles described herein have an average hydrodynamic diameter from between 50 nm to 200 nm.

In some embodiments, lipid nanoparticles described herein have a PDI from about 0.01 to about 0.3. In some embodiments, lipid nanoparticles described herein have a PDI that is about 0.01, 0.02, 0.03, 0.04, 0.05, 0.06, 0.07, 0.08, 0.09, 0.1, 0.15, 0.2, 0.25, 0.3, or any range having endpoints defined by any two of the aforementioned values. For example, in some embodiments, lipid nanoparticles described herein have a PDI from about 0.05 to about 0.2, about 0.06 to about 0.1, or about 0.07 to about 0.09.

In some embodiments, encapsulation efficiency of lipid nanoparticles described herein is from about 80% to about 100%. In some embodiments, encapsulation efficiency of lipid nanoparticles described herein is about 80%, 85%, 90%, 91%, 92%, 93%, 94%, 95%, 95.5%, 96%, 96.5%, 97%, 97.5%, 98%, 98.5%, 99%, 99.5%, 100%, or any range having endpoints defined by any two of the aforementioned values. For example, in some embodiments, encapsulation efficiency of lipid nanoparticles described herein is from about 90% to about 100%, about 95% to about 100%, about 95% to about 98%, or about 95.5% to about 97.5%. In some embodiments, encapsulation efficiency of lipid nanoparticles described herein is at least about 90%, 91%, 92%, 93%, 94%, 95%, 96%, 97%, 98%, or 99%.

In some embodiments, lipid nanoparticles described herein have a pKa from about 5 to about 9. In some embodiments, lipid nanoparticles described herein have a pKa that is about 5.0, 5.5, 6.0, 6.5, 7.0, 7.5, 8.0, 8.5, or any range having endpoints defined by any two of the aforementioned values. In some embodiments, lipid nanoparticles described herein have a pKa that is about 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, or any range having endpoints defined by any two of the aforementioned values.

The present invention provides for compositions, preparations, nanoparticles, and/or nanomaterials that comprise lipid nanoparticles. In some embodiments, a lipid nanoparticle preparation comprises about 30 mole percent to about 70 mole percent ionizable lipid, about 5 mole percent to about 25 mole percent phospholipid, about 25 mole percent to about 45 mole percent cholesterol, and about 0 mole percent to about 5 mole percent conjugate-linker lipid.

In some embodiments, a lipid nanoparticle preparation comprises about 45 mole percent ionizable lipid, about 9 mole percent phospholipid, about 44 mole percent cholesterol, and about 2 mole percent conjugate-linker lipid. In some embodiments, a lipid nanoparticle preparation comprises about 50 mole percent ionizable lipid, about 9 mole percent phospholipid, about 38 mole percent cholesterol, and about 3 mole percent conjugate-linker lipid.

In some embodiments, a lipid nanoparticle preparation comprises about 40 mole percent to about 60 mole percent ionizable lipid of any provided compound, about 5 mole percent to about 15 mole percent 1-2-distearoyl-sn-glycero-3-phosphocholine, about 1 mole percent to about 5 mole percent C14PEG2000, and about 30 mole percent to about 47 mole percent cholesterol, based on the total moles of these four ingredients.

In some embodiments, a lipid nanoparticle (LNP) preparation comprises a mass ratio of (ionizable lipid, cholesterol, lipid-PEG, and phospholipid):mRNA from about 2:1 and 50:1. In some embodiments, a LNP preparation comprises a mass ratio of (ionizable lipid, cholesterol, lipid-PEG, and phospholipid):mRNA of about 2:1, about 3:1, about 4:1, about 5:1, about 6:1, about 7:1, about 8:1, about 9:1, about 10:1, about 11:1, about 12:1, about 13:1, about 14:1, about 15:1, about 16:1, about 17:1, about 18:1, about 19:1, about 20:1, about 21:1, about 22:1, about 23:1, about 24:1, about 25:1, about 26:1, about 27:1, about 28:1, about 29:1, about 30:1, about 31:1, about 32:1, about 33:1, about 34:1, about 35:1, about 36:1, about 37:1, about 38:1, about 39:1, about 40:1, about 41:1, about 42:1, about 43:1, about 44:1, about 45:1, about 46:1, about 47:1, about 48:1, about 49:1, about 50:1. In some embodiments, a lipid nanoparticle (LNP) preparation comprises a mass ratio of (ionizable lipid, cholesterol, lipid-PEG, and phospholipid):mRNA of about 11.7:1 and 19:1.

In some embodiments, a lipid nanoparticle preparation comprises a mass ratio of (ionizable lipid, cholesterol, lipid-PEG, and phospholipid):siRNA from about 2:1 and 50:1. In some embodiments, a LNP preparation comprises a mass ratio of (ionizable lipid, cholesterol, lipid-PEG, and phospholipid):mRNA of about 2:1, about 3:1, about 4:1, about 5:1, about 6:1, about 7:1, about 8:1, about 9:1, about 10:1, about 11:1, about 12:1, about 13:1, about 14:1, about 15:1, about 16:1, about 17:1, about 18:1, about 19:1, about 20:1, about 21:1, about 22:1, about 23:1, about 24:1, about 25:1, about 26:1, about 27:1, about 28:1, about 29:1, about 30:1, about 31:1, about 32:1, about 33:1, about 34:1, about 35:1, about 36:1, about 37:1, about 38:1, about 39:1, about 40:1, about 41:1, about 42:1, about 43:1, about 44:1, about 45:1, about 46:1, about 47:1, about 48:1, about 49:1, about 50:1. In some embodiments, a lipid nanoparticle (LNP) preparation comprises a mass ratio of (ionizable lipid, cholesterol, lipid-PEG, and phospholipid):mRNA of about 11.7:1 and 19:1.

In some embodiments, an LNP preparation comprises one or more nucleic acids such as RNAs. In some embodiments, one or more nucleic acids (e.g., RNAs), lipids, and amounts thereof in an LNP preparation may be selected to provide a specific N/P ratio. The N/P ratio can be selected from about 1 to about 30. The N/P ratio can be selected from about 2 to about 12. In some embodiments, the N/P ratio is from about 0.1 to about 50. In some embodiments, the N/P ratio is from about 2 to about 8. In some embodiments, the N/P ratio is from about 2 to about 15, from about 2 to about 10, from about 2 to about 8, from about 2 to about 6, from about 3 to about 15, from about 3 to about 10, from about 3 to about 8, from about 3 to about 6, from about 4 to about 15, from about 4 to about 10, from about 4 to about 8, from about 4 to about 6, or from about 5 to about 7. In some embodiments, the N/P ratio is about 2, about 2.5, about 3, about 3.5, about 4, about 4.5, about 5, about 5.5, about 6, about 6.5, about 7, about 7.5, about 8, about 9, or about 10. In some embodiments, the N/P ratio is from about 4 to about 6. In some embodiments, the N/P ratio is about 4, about 4.5, about 5, about 5.5, or about 6.

As used herein, the "N/P ratio" is the molar ratio of ionizable (e.g., in the physiological pH range) nitrogen atoms in a lipid (or lipids) to phosphate groups in a nucleic acid molecular entity (or nucleic acid molecular entities), e.g., in a nanoparticle composition comprising a lipid component and an RNA. Ionizable nitrogen atoms can include, for example, nitrogen atoms that can be protonated at about pH 1, about pH 2, about pH 3, about pH 4, about pH 4.5, about pH 5, about pH 5.5, about pH 6, about pH 6.5, about pH 7, about pH 7.5, or about pH 8 or higher. The physiological pH range can include, for example, the pH range of different cellular compartments (such as organs, tissues, and cells) and bodily fluids (such as blood, CSF, gastric juice, milk, bile, saliva, tears, and urine). In certain specific embodiments, the physiological pH range refers to the pH range of blood in a mammal, for example, from about 7.35 to about 7.45. Similarly, for phosphate charge neutralizers that have one or more ionizable nitrogen atoms, the N/P ratio can refer to a molar ratio of ionizable nitrogen atoms in the phosphate charge neutralizer to the phosphate groups in a nucleic acid. In some embodiments, ionizable nitrogen atoms refer to those nitrogen atoms that are ionizable within a pH range between 5 and 14.

### Sterols

This disclosure describes compositions, preparations, nanoparticles, and nanomaterials containing one or more sterols.

In certain embodiments, the sterol component may be cholesterol, or a variant or derivative thereof. The cholesterol may be subject to modification. In some cases, the cholesterol is an oxidized cholesterol, while in others, it is an esterified cholesterol. It is contemplated that unmodified cholesterol may undergo enzymatic conversion to produce variants exhibiting side-chain or ring oxidation. The cholesterol may be oxidized at the beta-ring structure or the hydrocarbon tail structure. In some instances, the sterol is a phytosterol. Non-limiting examples of sterols suitable for use in the disclosed lipid nanoparticles include 25-hydroxycholesterol (25-OH), 20a-hydroxycholesterol (20α-OH), 27-hydroxycholesterol, 6-keto-5a-hydroxycholesterol, 7-ketocholesterol, 7β-hydroxycholesterol, 7a-hydroxycholesterol, 7B-25-dihydroxycholesterol, beta-sitosterol, stigmasterol, brassicasterol, campesterol, and combinations thereof. It is further contemplated that a side-chain oxidized cholesterol may improve cargo delivery compared to other cholesterol variants. In certain cases, the cholesterol is an unmodified cholesterol.

The LNP composition can comprise sterol in a range of approximately 20 mol percent to approximately 50 mol percent. In some versions, the LNP composition comprises roughly 38 mol percent sterol, roughly 38.5 mol percent sterol, or roughly 33.8 mol percent cholesterol.

### Conjugate-Linker Lipids

In various aspects, the present technology encompasses compositions, preparations, nanoparticles, and/or nanomaterials that incorporate one or more conjugate-linker lipids.

In certain embodiments, a conjugate-linker lipid may constitute or include a polyethylene glycol (PEG)-lipid or PEG-modified lipid. Such PEG or PEG-modified lipids may also be termed PEGylated lipids or PEG-lipids. The inclusion of a PEGylating lipid may serve to enhance the colloidal stability of lipid nanoparticles in vitro and extend their circulation time in vivo. In some instances, the PEGylation is reversible, with the PEG moiety being gradually released into blood circulation. Exemplary PEG-lipids include, but are not limited to, PEG conjugated to saturated or unsaturated alkyl chains having a length of C6-C20. Specific examples include PEG-modified phosphatidylethanolamines, PEG-modified phosphatidic acids, PEG-modified ceramides (PEG-CER), PEG-modified dialkylamines, PEG-modified diacylglycerols (PEG-DAG), PEG-modified dialkylglycerols, and mixtures thereof. For instance, in some embodiments, a PEG lipid may be PEG-c-DOMG, PEG-DMG, PEG-DLPE, PEG-DMPE, PEG-DPPE, PEG-DSG or a PEG-DSPE lipid.

In some embodiments, a conjugate-linker lipid comprises a polyethylene glycol lipid. In some embodiments, the conjugate-linker lipid comprises DiMystyrlGlycerol (DMG), 1,2-Dipalmitoyl-rac-glycerol, methoxypolyethylene Glycol (DPG-PEG), or 1,2-Distearoyl-rac-glycero-3-methylpolyoxyethylene (DSG - PEG). In certain embodiments, a conjugate-linker lipid has an average molecular mass ranging from about 500 Da to about 5000 Da. In some embodiments, a conjugate-linker lipid has an average molecular mass of about 2000 Da. The LNP composition may include conjugate-linker lipid ranging from approximately 0 mol percent to approximately 5 mol percent. In certain versions, the LNP composition comprises roughly 1.5 mol percent conjugate-linker lipid or roughly 3 mol percent conjugate-linker lipid.

### Phospholipids

The technology described herein also relates to compositions, preparations, nanoparticles, and/or nanomaterials that comprise one or more phospholipids. In some embodiments, the present disclosure describes compositions, preparations, nanoparticles, and/or nanomaterials that comprise one or more (poly)unsaturated lipids.

In certain instances, one or more phospholipids may self-assemble into one or more lipid bilayers. In some versions, one or more phospholipids may include a phospholipid moiety, one or more fatty acid moieties, or both a phospholipid moiety and one or more fatty acid moieties. The phospholipid moiety can include phosphatidyl choline, phosphatidyl ethanolamine, phosphatidyl glycerol, phosphatidyl serine, phosphatidic acid, 2-lysophosphatidyl choline, and sphingomyelin, without limitation. The fatty acid moiety can include lauric acid, myristic acid, myristoleic acid, palmitic acid, palmitoleic acid, stearic acid, oleic acid, linoleic acid, alphalinolenic acid, erucic acid, phytanic acid, arachidic acid, arachidonic acid, eicosapentaenoic acid, behenic acid, docosapentaenoic acid, and docosahexaenoic acid, among others. It is contemplated that non-natural species may be utilized, including natural species with modifications and substitutions such as branching, oxidation, cyclization, and alkynes. For example, a phospholipid may be functionalized with or cross-linked to one or more alkynes (e.g., an alkenyl group in which one or more double bonds is replaced with a triple bond). Under appropriate reaction conditions, an alkyne group may undergo a copper-catalyzed cycloaddition upon exposure to an azide. Such reactions may be useful in functionalizing a lipid bilayer of a nanoparticle composition to facilitate membrane permeation or cellular recognition or in conjugating a nanoparticle composition to a useful component such as a targeting or imaging moiety (e.g., a dye).

Exemplary phospholipids include, but are not limited to 1,2-distearoyl-snglycero-3-phosphocholine (DSPC), 1,2-dioleoyl-sn-glycero-3-phosphoethanolamine (DOPE), 1,2-dilinoleoyl-sn-glycero-3-phosphocholine (DLPC), 1,2-dimyristoyl-sn-glycerophosphocholine (DMPC), 1,2-dioleoyl-sn-glycero-3-phosphocholine (DOPC), 1,2-dipalmitoyl-sn-glycero-3-phosphocholine (DPPC), 1,2-diundecanoyl-sn-glycerophosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoy 1-sn-glycero-3-phosphocholine (OChemsPC), 1-hexadecyl snglycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1 -glycerol) sodium salt (DOPG), dipalmitoylphosphatidylglycerol (DPPG), palmitoyloleoylphosphatidylethanolamine (POPE), distearoyl-phosphatidyl-ethanolamine (DSPE), dipalmitoyl phosphatidyl ethanolamine (DPPE), dimyristoylphosphoethanolamine (DMPE), 1-stearoyl-2-oleoyl-phosphatidy ethanolamine (SOPE), 1-stearoyl-2 oleoylphosphatidylcholine (SOPC), sphingomyelin, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine (LPE), or combinations thereof. In some embodiments, a phospholipid is DSPC. In some embodiments, a phospholipid is DMPC.

### Conjugate-Linker Lipids

The technology detailed herein also contemplates compositions, preparations, nanoparticles, and/or nanomaterials that include at least one conjugate-linker lipid.

In certain embodiments, the conjugate-linker lipid can be a polyethylene glycol (PEG)-lipid or a lipid modified with PEG. Those skilled in the art will recognize that PEGylated lipids or PEG-lipids are other terms to describe such PEG or PEG-modified lipids. Incorporation of a PEG-comprising lipid may enhance the colloidal stability of a lipid nanoparticle in vitro and/or increase its circulation time in vivo. In some instances, the PEGylation is designed to be reversible, such that the PEG moiety is gradually released into circulation. The PEG-lipids may include, but are not limited to, PEG conjugated to saturated or unsaturated alkyl chains ranging in length from six to twenty carbon atoms. Specific examples include PEG-modified phosphatidylethanolamines, PEG-modified phosphatidic acids, PEG-modified ceramides (PEG-CER), PEG-modified dialkylamines, PEG-modified diacylglycerols (PEG-DAG), PEG-modified dialkylglycerols, and combinations thereof. By way of example, a PEG lipid may be PEG-c-DOMG, PEG-DMG, PEG-DLPE, PEG-DMPE, PEG-DPPE, PEG-DSG or a PEG-DSPE lipid.

In some embodiments, the conjugate-linker lipid comprises a polyethylene glycol lipid. As an example, the conjugate-linker lipid may comprise DiMystyrlGlycerol (DMG), 1,2-Dipalmitoyl-rac-glycerol, methoxypolyethylene Glycol (DPG-PEG), or 1,2-Distearoyl-rac-glycero-3-methylpolyoxyethylene (DSG - PEG). In certain embodiments, a conjugate-linker lipid has a molecular mass averaging from about 500 Daltons to about 5000 Daltons. In other cases, a conjugate-linker lipid has a molecular mass averaging about 2000 Daltons. The LNP composition may comprise conjugate-linker lipid from about 0 mole percent to about 5 mole percent. In some specific embodiments, the LNP composition comprises about 1.5 mole percent conjugate-linker lipid, or alternatively about 3 mole percent conjugate-linker lipid.

### Diameter

The present technology also relates to compositions, preparations, nanoparticles, and/or nanomaterials that have an average hydrodynamic diameter ranging from approximately 30 to approximately 220 nm. In some embodiments, the compositions, preparations, nanoparticles, and/or nanomaterials exhibit an average hydrodynamic diameter of about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, 150 nm, 155 nm, 160 nm, 165 nm, 170 nm, 175 nm, 180 nm, 185 nm, 190 nm, 195 nm, 200 nm, 205 nm, 210 nm, 215 nm, 220 nm, or any range defined by any two of the aforementioned values. In certain instances, the compositions, preparations, nanoparticles, and/or nanomaterials possess an average hydrodynamic diameter between approximately 50 nm and approximately 200 nm.

Lipid nanoparticles may have an average hydrodynamic diameter within a range of approximately 30 to approximately 220 nm. In some versions, the lipid nanoparticles possess an average hydrodynamic diameter of roughly 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, 150 nm, 155 nm, 160 nm, 165 nm, 170 nm, 175 nm, 180 nm, 185 nm, 190 nm, 195 nm, 200 nm, 205 nm, 210 nm, 215 nm, 220 nm, or any range with endpoints defined by any two of the aforementioned values. For example, lipid nanoparticles may exhibit an average hydrodynamic diameter ranging from approximately 50 nm to approximately 200 nm.

### Polydispersity

The present technology further pertains to compositions, preparations, nanoparticles, and/or nanomaterials that exhibit a polydispersity index (PDI) ranging from approximately 0.01 to approximately 0.3. In some embodiments, the compositions, preparations, nanoparticles, and/or nanomaterials may exhibit a PDI of about 0.01, 0.02, 0.03, 0.04, 0.05, 0.06, 0.07, 0.08, 0.09, 0.1, 0.15, 0.2, 0.25, 0.3, or any range with endpoints defined by two of the aforementioned values. For instance, in certain embodiments, the compositions, preparations, nanoparticles, and/or nanomaterials have a PDI ranging from approximately 0.05 to approximately 0.2, approximately 0.06 to approximately 0.1, or approximately 0.07 to approximately 0.09.

Lipid nanoparticles may have a PDI ranging from roughly 0.01 to roughly 0.3. In certain versions, the lipid nanoparticles exhibit a PDI of about 0.01, 0.02, 0.03, 0.04, 0.05, 0.06, 0.07, 0.08, 0.09, 0.1, 0.15, 0.2, 0.25, 0.3, or a range defined by two of the aforementioned values. For example, lipid nanoparticles may have a PDI ranging from about 0.05 to 0.2, about 0.06 to 0.1, or about 0.07 to 0.09.

### Encapsulation Efficiency

The technology described herein also encompasses compositions, preparations, nanoparticles, and/or nanomaterials, wherein the encapsulation efficiency of the compositions, preparations, nanoparticles, and/or nanomaterials ranges from approximately 80% to approximately 100%. In some versions, the encapsulation efficiency of the compositions, preparations, nanoparticles, and/or nanomaterials is approximately 80%, 85%, 90%, 91%, 92%, 93%, 94%, 95%, 95.5%, 96%, 96.5%, 97%, 97.5%, 98%, 98.5%, 99%, 99.5%, 100%, or any range using endpoints defined by the aforementioned values. In some embodiments, the encapsulation efficiency of the compositions, preparations, nanoparticles, and/or nanomaterials is in a range of about 90% to about 100%, about 95% to about 100%, about 95% to about 98%, or about 95.5% to about 97.5%. The encapsulation efficiency of the compositions, preparations, nanoparticles, and/or nanomaterials is at least about 90%, 91%, 92%, 93%, 94%, 95%, 96%, 97%, 98%, or 99%.

The encapsulation efficiency of lipid nanoparticles can range from approximately 80% to approximately 100%. The encapsulation efficiency of lipid

### pKa

Furthermore, the technology described herein relates to compositions, preparations, nanoparticles, and/or nanomaterials that exhibit a pKa ranging from approximately 5 to approximately 9. In some embodiments, the compositions, preparations, nanoparticles, and/or nanomaterials possess a pKa of around 5.0, 5.5, 6.0, 6.5, 7.0, 7.5, 8.0, 8.5, or any range having endpoints defined by any two of the aforementioned values. In certain instances, the compositions, preparations, nanoparticles, and/or nanomaterials have a pKa of roughly 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, or any range with endpoints defined by any two of the aforementioned values.

Lipid nanoparticles may have a pKa ranging from roughly 5 to roughly 9. In some versions, lipid nanoparticles have a pKa of around 5.0, 5.5, 6.0, 6.5, 7.0, 7.5, 8.0, 8.5, or any range having endpoints defined by any two of the aforementioned values. In certain embodiments, lipid nanoparticles possess a pKa of approximately 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, or any range having endpoints defined by any two of the aforementioned values.

### Exemplary LNP Preparations

The present technology provides for compositions, preparations, nanoparticles, and/or nanomaterials that comprise lipid nanoparticles. In some embodiments, a lipid nanoparticle preparation comprises approximately 30 mole percent to approximately 70 mole percent ionizable lipid, approximately 5 mole percent to approximately 25 mole percent phospholipid, approximately 25 mole percent to approximately 45 mole percent cholesterol, and approximately 0 mole percent to approximately 5 mole percent conjugate-linker lipid.

In specific embodiments, a lipid nanoparticle preparation may comprise roughly 45 mole percent ionizable lipid, roughly 9 mole percent phospholipid, roughly 44 mole percent cholesterol, and roughly 2 mole percent conjugate-linker lipid. Alternatively, a lipid nanoparticle preparation may comprise roughly 50 mole percent ionizable lipid, roughly 9 mole percent phospholipid, roughly 38 mole percent cholesterol, and roughly 3 mole percent conjugate-linker lipid.

In some embodiments, a lipid nanoparticle preparation comprises approximately 40 mole percent to approximately 60 mole percent ionizable lipid of any provided compound, approximately 5 mole percent to approximately 15 mole percent 1-2-distearoyl-sn-glycero-3-phosphocholine, approximately 1 mole percent to approximately 5 mole percent C14PEG2000, and approximately 30 mole percent to approximately 47 mole percent cholesterol, based on the total moles of these four ingredients.

In some embodiments, a lipid nanoparticle (LNP) preparation comprises a mass ratio of (ionizable lipid, cholesterol, lipid-PEG, and phospholipid):mRNA from approximately 2:1 and 50:1. In some embodiments, a LNP preparation comprises a mass ratio of (ionizable lipid, cholesterol, lipid-PEG, and phospholipid):mRNA of about 2:1, about 3:1, about 4:1, about 5:1, about 6:1, about 7:1, about 8:1, about 9:1, about 10:1, about 11:1, about 12:1, about 13:1, about 14:1, about 15:1, about 16:1, about 17:1, about 18:1, about 19:1, about 20:1, about 21:1, about 22:1, about 23:1, about 24:1, about 25:1, about 26:1, about 27:1, about 28:1, about 29:1, about 30:1, about 31:1, about 32:1, about 33:1, about 34:1, about 35:1, about 36:1, about 37:1, about 38:1, about 39:1, about 40:1, about 41:1, about 42:1, about 43:1, about 44:1, about 45:1, about 46:1, about 47:1, about 48:1, about 49:1, about 50:1. In some embodiments, a lipid nanoparticle (LNP) preparation comprises a mass ratio of (ionizable lipid, cholesterol, lipid-PEG, and phospholipid):mRNA of about 11.7:1 and 19:1.

In some embodiments, a lipid nanoparticle preparation comprises a mass ratio of (ionizable lipid, cholesterol, lipid-PEG, and phospholipid):siRNA from about 2:1 and 50:1. In some embodiments, a LNP preparation comprises a mass ratio of (ionizable lipid, cholesterol, lipid-PEG, and phospholipid):mRNA of about 2:1, about 3:1, about 4:1, about 5:1, about 6:1, about 7:1, about 8:1, about 9:1, about 10:1, about 11:1, about 12:1, about 13:1, about 14:1, about 15:1, about 16:1, about 17:1, about 18:1, about 19:1, about 20:1, about 21:1, about 22:1, about 23:1, about 24:1, about 25:1, about 26:1, about 27:1, about 28:1, about 29:1, about 30:1, about 31:1, about 32:1, about 33:1, about 34:1, about 35:1, about 36:1, about 37:1, about 38:1, about 39:1, about 40:1, about 41:1, about 42:1, about 43:1, about 44:1, about 45:1, about 46:1, about 47:1, about 48:1, about 49:1, about 50:1. In some embodiments, a lipid nanoparticle (LNP) preparation comprises a mass ratio of (ionizable lipid, cholesterol, lipid-PEG, and phospholipid):mRNA of about 11.7:1 and 19:1.

In some embodiments, an LNP preparation comprises one or more nucleic acids such as RNAs. In some embodiments, one or more nucleic acids (e.g., RNAs), lipids, and amounts thereof in an LNP preparation may be selected to provide a specific N/P ratio. The N/P ratio can be selected from about 1 to about 30. The N/P ratio can be selected from about 2 to about 12. In some embodiments, the N/P ratio is from about 0.1 to about 50. In some embodiments, the N/P ratio is from about 2 to about 8. In some embodiments, the N/P ratio is from about 2 to about 15, from about 2 to about 10, from about 2 to about 8, from about 2 to about 6, from about 3 to about 15, from about 3 to about 10, from about 3 to about 8, from about 3 to about 6, from about 4 to about 15, from about 4 to about 10, from about 4 to about 8, from about 4 to about 6, or from about 5 to about 7. In some embodiments, the N/P ratio is about 2, about 2.5, about 3, about 3.5, about 4, about 4.5, about 5, about 5.5, about 6, about 6.5, about 7, about 7.5, about 8, about 9, or about 10. In some embodiments, the N/P ratio is from about 4 to about 6. In some embodiments, the N/P ratio is about 4, about 4.5, about 5, about 5.5, or about 6.

As used herein, the "N/P ratio" is the molar ratio of ionizable (e.g., in the physiological pH range) nitrogen atoms in a lipid (or lipids) to phosphate groups in a nucleic acid molecular entity (or nucleic acid molecular entities), e.g., in a nanoparticle composition comprising a lipid component and an RNA. Ionizable nitrogen atoms can include, for example, nitrogen atoms that can be protonated at about pH 1, about pH 2, about pH 3, about pH 4, about pH 4.5, about pH 5, about pH 5.5, about pH 6, about pH 6.5, about pH 7, about pH 7.5, or about pH 8 or higher. The physiological pH range can include, for example, the pH range of different cellular compartments (such as organs, tissues, and cells) and bodily fluids (such as blood, CSF, gastric juice, milk, bile, saliva, tears, and urine). In certain specific embodiments, the physiological pH range refers to the pH range of blood in a mammal, for example, from about 7.35 to about 7.45. Similarly, for phosphate charge neutralizers that have one or more ionizable nitrogen atoms, the N/P ratio can refer to a molar ratio of ionizable nitrogen atoms in the phosphate charge neutralizer to the phosphate groups in a nucleic acid. In some embodiments, ionizable nitrogen atoms refer to those nitrogen atoms that are ionizable within a pH range between 5 and 14.

### Pharmaceutical Compositions

The present technology also provides for compositions, preparations, nanoparticles, and/or nanomaterials that comprise pharmaceutical compositions. Among other things, in some embodiments, pharmaceutical compositions comprise lipid nanoparticles and lipid nanoparticle preparations described herein. For example, in some embodiments, lipid nanoparticles and lipid nanoparticle preparations described herein can be formulated in whole or in part as pharmaceutical compositions.

In some embodiments, pharmaceutical compositions may include one or more nanoparticle compositions described herein. For example, a pharmaceutical composition may comprise one or more nanoparticle compositions including one or more different therapeutic and/or prophylactics including but not limited to one or more nucleic acids of different types or encode different agents. In some embodiments, a pharmaceutical composition comprises one or more pharmaceutically acceptable excipients or accessory ingredients including but not limited to a pharmaceutically acceptable carrier.

A pharmaceutical composition may be administered to a subject. In some embodiments, a pharmaceutical composition is administered as described herein. In some in vivo approaches, the nanoparticle compositions disclosed herein are administered to a subject in a therapeutically effective amount as described herein.

In some embodiments, the ordinary skilled worker, considering the therapeutic context, age, and general health of the recipient, will be able to devise an appropriate dosage level and dosing regimen using the pharmaceutical compositions described herein for treatment of various conditions in various patients. For example, in some embodiments, a selected dosage depends upon the desired therapeutic effect, on the route of administration, and on the duration of the treatment desired. In some embodiments, generally dosage levels of about 0.001 mg to about 5 mg of nucleic acid per kg of body weight are administered each dosage to mammals. More specifically, in some embodiments, a preferential dose for nucleic acids within the disclosed nanoparticles is about 0.1 mg / kg to about 1.0 mg/kg. For the disclosed nanoparticles, generally dosage levels of about 0.2 mg to about 100 mg of four components (ionizable lipid, cholesterol, conjugate-linker conjugate, and phospholipid) / kg of body weight are administered to mammals. More specifically, in some embodiments, a preferential dose of the disclosed nanoparticles is about 0.5 mg / kg to about 5 mg / kg of the four components / kg of body weight.

In some embodiments, a pharmaceutical composition described herein is administered locally, for example by injection directly into a site to be treated. Typically, the injection causes an increased localized concentration of the composition which is greater than that which can be achieved by systemic administration. In some embodiments, a pharmaceutical composition described herein can be combined with a matrix as described herein to assist in creating an increased localized concentration of the polypeptide compositions by reducing the passive diffusion of the polypeptides out of the site to be treated.

### Cargo

In certain aspects, the present invention provides for compositions, preparations, nanoparticles, and/or nanomaterials that comprise cargo as described herein. The compositions, preparations, nanoparticles, and/or nanomaterials may include a therapeutic or prophylactic agent for delivery to a subject. In some embodiments, a therapeutic or prophylactic agent is encapsulated by a lipid nanoparticle. In certain cases, a lipid nanoparticle is loaded with one or more nucleic acids.

### Therapeutic and/or Prophylactic Agents

Cargo delivered via a LNP composition may be a biologically active agent. In some embodiments, the cargo is or comprises one or more biologically active agents, such as mRNA, guide RNA (gRNA), nucleic acid, RNA-guided DNA-binding agent, expression vector, template nucleic acid, antibody (e.g., monoclonal, chimeric, humanized, nanobody, and fragments thereof etc.), cholesterol, hormone, peptide, protein, chemotherapeutic and other types of antineoplastic agent, low molecular weight drug, vitamin, co-factor, nucleoside, nucleotide, oligonucleotide, enzymatic nucleic acid, antisense nucleic acid, triplex forming oligonucleotide, antisense DNA or RNA composition, chimeric DNA:RNA composition, allozyme, aptamer, ribozyme, decoys and analogs thereof, plasmid and other types of vectors, and small nucleic acid molecule, RNAi agent, short interfering nucleic acid (siNA), short interfering RNA (siRNA), double-stranded RNA (dsRNA), micro-RNA (miRNA), short hairpin RNA (shRNA) and "self-replicating RNA" (encoding a replicase enzyme activity and capable of directing its own replication or amplification in vivo) molecules, peptide nucleic acid (PNA), a locked nucleic acid ribonucleotide (LNA), morpholino nucleotide, threose nucleic acid (TNA), glycol nucleic acid (GNA), sisiRNA (small internally segmented interfering RNA), and iRNA (asymmetrical interfering RNA). The above list of biologically active agents is exemplary only, and is not intended to be limiting. Such compounds may be purified or partially purified, and may be naturally occurring or synthetic, and may be chemically modified.

Cargo delivered via a LNP composition may be an RNA, such as an mRNA molecule encoding a protein of interest. For example, in some embodiments, an mRNA for expressing a protein such as green fluorescent protein (GFP), an RNA-guided DNA-binding agent, or a Cas nuclease is described herein. LNP compositions that include a Cas nuclease mRNA, for example a Class 2 Cas nuclease mRNA that allows for expression in a cell of a Class 2 Cas nuclease such as a Cas9 or Cpf1 protein are provided. Further, cargo may contain one or more guide RNAs or nucleic acids encoding guide RNAs. A template nucleic acid, e.g., for repair or recombination, may also be included in the composition or a template nucleic acid may be used in the methods described herein. In some embodiments, cargo comprises an mRNA that encodes a Streptococcus pyogenes Cas9, optionally and an S. pyogenes gRNA. In some embodiments, cargo comprises an mRNA that encodes a Neisseria meningitidis Cas9, optionally and an nme gRNA.

### CRISPR/Cas Cargo

In some embodiments, the disclosed compositions, preparations, nanoparticles, and/or nanomaterials comprise an mRNA encoding an RNA-guided DNA-binding agent, such as a Cas nuclease. In particular embodiments, the disclosed compositions, preparations, nanoparticles, and/or nanomaterials comprise an mRNA encoding a Class 2 Cas nuclease, such as S. *pyogenes* Cas9.

An "RNA-guided DNA binding agent" refers to a polypeptide or complex of polypeptides exhibiting RNA and DNA binding activity, or a DNA-binding subunit of such a complex, characterized in that the DNA binding activity exhibits sequence specificity contingent upon the RNA sequence. Exemplary RNA-guided DNA binding agents encompass Cas cleavases/nickases and inactivated forms thereof ("dCas DNA binding agents"). "Cas nuclease" encompasses Cas cleavases, Cas nickases, and dCas DNA binding agents. Cas cleavases/nickases and dCas DNA binding agents include a Csm or Cmr complex of a type III CRISPR system, the Cas10, Csml, or Cmr2 subunit thereof, a Cascade complex of a type I CRISPR system, the Cas3 subunit thereof, and Class 2 Cas nucleases. A "Class 2 Cas nuclease" is a single chain polypeptide with RNA-guided DNA binding activity. Class 2 Cas nucleases include Class 2 Cas cleavases/nickases (e.g., H840A, D10A, or N863A variants), which further have RNA-guided DNA cleavases or nickase activity, and Class 2 dCas DNA binding agents, in which cleavase/nickase activity is inactivated. Class 2 Cas nucleases include, for example, Cas9, Cpf1, C2c1, C2c2, C2c3, HF Cas9 (e.g., N497A, R661A, Q695A, Q926A variants), HypaCas9 (e.g., N692A, M694A, Q695A, H698A variants), eSPCas9(1.0) (e.g., K810A, K1003A, R1060A variants), and eSPCas9(1.1) (e.g., K848A, K1003A, R1060A variants) proteins and modifications thereof. Cpfl protein, Zetsche et al., Cell, 163: 1-13 (2015), is homologous to Cas9, and contains a RuvC-like nuclease domain. Cpfl sequences of Zetsche are incorporated by reference in their entirety herein.

In some embodiments the CRISPR/Cas Cargo comprises a Type V nuclease. In yet other embodiments the CRISPR/Cas Cargo comprises a Type V nuclease of the B-GEn family as disclosed in WO2022258753A1.

"Ribonucleoprotein" (RNP) or "RNP complex" refers to a guide RNA together with an RNA-guided DNA binding agent, such as a Cas nuclease, e.g., a Cas cleavase, Cas nickase, or dCas DNA binding agent (e.g., Cas9). In some embodiments, the guide RNA guides the RNA-guided DNA binding agent such as Cas9 to a target sequence, and the guide RNA hybridizes with and the agent binds to the target sequence; in cases where the agent is a cleavase or nickase, binding can be followed by cleaving or nicking.

In some embodiments, cargo for a LNP composition includes at least one guide RNA comprising guide sequences that direct an RNA-guided DNA binding agent, which can be a nuclease (e.g., a Cas nuclease such as Cas9), to a target DNA. gRNA may guide the Cas nuclease or Class 2 Cas nuclease to a target sequence on a target nucleic acid molecule. In some embodiments, a gRNA binds with and provides specificity of cleavage by a Class 2 Cas nuclease. In some embodiments, a gRNA and a Cas nuclease may form a ribonucleoprotein (RNP), e.g., a CRISPR/Cas complex such as a CRISPR/Cas9 complex. In some embodiments, a CRISPR/Cas complex may be a Type-II CRISPR/Cas9 complex. In some embodiments, a CRISPR/Cas complex may be a Type-V CRISPR/Cas complex, such as a Cpfl/guide RNA complex. Cas nucleases and cognate gRNAs may be paired. A gRNA scaffold structures that pair with each Class 2 Cas nuclease vary with the specific CRISPR/Cas system.

"Guide RNA", "gRNA", and simply "guide" are used herein interchangeably to refer to either a crRNA (also known as CRISPR RNA), or the combination of a crRNA and a trRNA (also known as tracrRNA). Guide RNAs can include modified RNAs as described herein. The crRNA and trRNA may be associated as a single RNA molecule (single guide RNA, sgRNA) or in two separate RNA molecules (dual guide RNA, dgRNA). "Guide RNA" or "gRNA" refers to each type. trRNA may be a naturally-occurring sequence, or a trRNA sequence with modifications or variations compared to naturally-occurring sequences.

A "guide sequence" refers to a sequence within a guide RNA that is complementary to a target sequence and functions to direct a guide RNA to a target sequence for binding or modification (e.g., cleavage) by an RNA-guided DNA binding agent. A "guide sequence" may also be referred to as a "targeting sequence," or a "spacer sequence." A guide sequence can be 20 base pairs in length, e.g., in the case of Streptococcus pyogenes (i.e., Spy Cas9) and related Cas9 homologs/orthologs. Shorter or longer sequences can also be used as guides, e.g., 15-, 16-, 17-, 18-, 19-, 21-, 22-, 23-, 24-, or 25-nucleotides in length. In some embodiments, a target sequence is in a gene or on a chromosome, for example, and is complementary to a guide sequence. In some embodiments, a degree of complementarity or identity between a guide sequence and its corresponding target sequence may be about or at least 75%, 80%, 85%, 90%, 95%, 96%, 97%, 98%, 99%, or 100%. In some embodiments, a guide sequence and the target region may be 100% complementary or identical over a region of at least 15, 16, 17, 18, 19, or 20 contiguous nucleotides. In other embodiments, a guide sequence and a target region may contain at least one mismatch. For example, a guide sequence and a target sequence may contain 1, 2, 3, or 4 mismatches, where the total length of the target sequence is at least 17, 18, 19, 20 or more base pairs. In some embodiments, a guide sequence and a target region may contain 1-4 mismatches where a guide sequence comprises at least 17, 18, 19, 20 or more nucleotides. In some embodiments, a guide sequence and the target region may contain 1, 2, 3, or 4 mismatches where the guide sequence comprises 20 nucleotides.

Target sequences for RNA-guided DNA binding proteins, such as Cas proteins, encompass both the sense and antisense strands of genomic DNA-namely, the provided sequence and its reverse complement-since a nucleic acid substrate for a Cas protein is double-stranded. Consequently, when a guide sequence is described as "complementary to a target sequence," it should be understood that the guide sequence may facilitate the binding of a guide RNA to the reverse complement of that target sequence. Therefore, in some cases, if the guide sequence binds to the reverse complement of the target sequence, the guide sequence will be identical to specific nucleotides of the target sequence (excluding the PAM) with the exception of substituting U for T in the guide sequence.

The length of the targeting sequence may depend on the CRISPR/Cas system and components used. For example, different Class 2 Cas nucleases from different bacterial species have varying optimal targeting sequence lengths. Accordingly, the targeting sequence may comprise 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more than 50 nucleotides in length. In some embodiments, the targeting sequence length is 0, 1, 2, 3, 4, or 5 nucleotides longer or shorter than the guide sequence of a naturally-occurring nucleotide sequence.

In certain embodiments, a Cas nuclease and gRNA scaffold will be derived from the same CRISPR/Cas system. In some embodiments, a targeting sequence may comprise or consist of 18-24 nucleotides. In some embodiments, a targeting sequence may comprise or consist of 19-21 nucleotides. In some embodiments, the targeting sequence may comprise or consist of 20 nucleotides.

In some embodiments, a sgRNA is a "Cas9 sgRNA" capable of mediating RNA-guided DNA cleavage by a Cas9 protein. In some embodiments, a sgRNA is a "Cpfl sgRNA" capable of mediating RNA-guided DNA cleavage by a Cpfl protein. In some embodiments, a gRNA comprises a crRNA and tracr RNA sufficient for forming an active complex with a Cas9 protein and mediating RNA-guided DNA cleavage. In some embodiments, a gRNA comprises a crRNA sufficient for forming an active complex with a Cpfl protein and mediating RNA-guided DNA cleavage. See Zetsche 2015.

### mRNA

In certain embodiments, the disclosed compositions, preparations, nanoparticles, and/or nanomaterials comprise an mRNA comprising an open reading frame (ORF) encoding an RNA-guided DNA binding agent, such as a Cas nuclease, or Class 2 Cas nuclease as described herein. In some embodiments, an mRNA comprising an ORF encoding an RNA-guided DNA binding agent, such as a Cas nuclease or Class 2 Cas nuclease, is provided, used, or administered. An mRNA may comprise one or more of a 5' cap, a 5' untranslated region (UTR), a 3' UTRs, and a polyadenine tail. The mRNA may comprise a modified open reading frame, for example to encode a nuclear localization sequence or to use alternate codons to encode the protein.

mRNA in the disclosed compositions, preparations, nanoparticles, and/or nanomaterials may encode, for example, a secreted hormone, enzyme, receptor, polypeptide, peptide or other protein of interest that is normally secreted. In one embodiment of the invention, the mRNA may optionally have chemical or biological modifications which, for example, improve the stability and/or half-life of such mRNA or which improve or otherwise facilitate protein production. In addition, suitable modifications include alterations in one or more nucleotides of a codon such that the codon encodes the same amino acid but is more stable than the codon found in the wild-type version of the mRNA. For example, an inverse relationship between the stability of RNA and a higher number cytidines (C's) and/or uridines (U's) residues has been demonstrated, and RNA devoid of C and U residues have been found to be stable to most RNases (Heidenreich, et al. J Biol Chem 269, 2131-8 (1994), the disclosure of which is hereby incorporated by reference herein in its entirety). In some embodiments, the number of C and/or U residues in an mRNA sequence is reduced. In another embodiment, the number of C and/or U residues is reduced by substitution of one codon encoding a particular amino acid for another codon encoding the same or a related amino acid. Contemplated modifications to the mRNA nucleic acids of the present invention also include the incorporation of pseudouridines. The incorporation of pseudouridines into the mRNA nucleic acids of the present invention may enhance stability and translational capacity, as well as diminishing immunogenicity in vivo. Furthermore, various modifications to the mRNA of the present invention may be performed by methods readily known to one or ordinary skill in the art.

The constraints on reducing the number of C and U residues in a sequence will likely be greater within the coding region of an mRNA, compared to an untranslated region, (i.e., it will likely not be possible to eliminate all of the C and U residues present in the message while still retaining the ability of the message to encode the desired amino acid sequence). The degeneracy of the genetic code, however presents an opportunity to allow the number of C and/or U residues that are present in the sequence to be reduced, while maintaining the same coding capacity (i.e., depending on which amino acid is encoded by a codon, several different possibilities for modification of RNA sequences may be possible).

The term modification also includes, for example, the incorporation of non-nucleotide linkages or modified nucleotides into the mRNA sequences of the present invention (e.g., modifications to one or both the 3' and 5' ends of an mRNA molecule encoding a functional secreted protein or enzyme). Such modifications include the addition of bases to an mRNA sequence (e.g., the inclusion of a poly A tail or a longer poly A tail), the alteration of the 3' UTR or the 5' UTR, complexing the mRNA with an agent (e.g., a protein or a complementary nucleic acid molecule), and inclusion of elements which change the structure of an mRNA molecule (e.g., which form secondary structures).

The poly A tail is thought to stabilize natural messengers. Therefore, in one embodiment a long poly A tail can be added to an mRNA molecule thus rendering the mRNA more stable. Poly A tails can be added using a variety of art-recognized techniques. For example, long poly A tails can be added to synthetic or in vitro transcribed mRNA using poly A polymerase. A transcription vector can also encode long poly A tails. In addition, poly A tails can be added by transcription directly from PCR products. In one embodiment, the length of the poly A tail is at least about 90, 200, 300, 400 at least 500 nucleotides. In one embodiment, the length of the poly A tail is adjusted to control the stability of a modified mRNA molecule of the invention and, thus, the transcription of protein. For example, since the length of the poly A tail can influence the half-life of an mRNA molecule, the length of the poly A tail can be adjusted to modify the level of resistance of the mRNA to nucleases and thereby control the time course of protein expression in a cell. In one embodiment, the stabilized mRNA molecules are sufficiently resistant to in vivo degradation (e.g., by nucleases), such that they may be delivered to the target cell without a transfer vehicle.

In some embodiment embodiments, an mRNA can be modified by the incorporation 3' and/or 5' untranslated (UTR) sequences which are not naturally found in the wild-type mRNA. In one embodiment, 3' and/or 5' flanking sequence which naturally flanks an mRNA and encodes a second, unrelated protein can be incorporated into the nucleotide sequence of an mRNA molecule encoding a therapeutic or functional protein in order to modify it. For example, 3' or 5' sequences from mRNA molecules which are stable (e.g., globin, actin, GAPDH, tubulin, histone, or citric acid cycle enzymes) can be incorporated into the 3' and/or 5' region of a sense mRNA nucleic acid molecule to increase the stability of the sense mRNA molecule.

### Template Nucleic Acid

The compositions, preparations, nanoparticles, and/or methods described herein may include a template nucleic acid. A template can be used to modify or insert a nucleic acid sequence at or near a target site for an RNA-guided DNA binding protein such as a Cas nuclease, e.g., a Class 2 Cas nuclease. In some instances, the methods involve introducing a template to the cell. A single template may be provided, or two or more templates may be provided to edit multiple target sites. For example, different templates may be used to edit a single gene in a cell, or two different genes in a cell.

A template may be utilized in homologous recombination, resulting in the integration of the template sequence or part of it into the target nucleic acid molecule. Homology-directed repair involves DNA strand invasion at the cleavage site in the nucleic acid, potentially incorporating the template sequence into the edited target molecule. Gene editing mediated by non-homologous end joining may also involve a template sequence, regardless of similarity to the nucleic acid sequence near the cleavage site. Templates may include flanking inverted terminal repeat (ITR) sequences.

The template sequence might be endogenous to the target cell, referring to native sequences, or exogenous, indicating sequences not native to the cell or found in different genomic locations. Endogenous sequences might be genomic, chromosomal, extrachromosomal, or plasmid sequences. Templates may consist of ssDNA or dsDNA with flanking ITR sequences and can be provided as vectors, plasmids, minicircles, nanocircles, or PCR products.

Nucleic acids may undergo purification using precipitation methods (e.g., LiCl precipitation, alcohol precipitation), chromatography-based methods (e.g., HPLC-based methods), or both. Tangential flow filtration (TFF) may also be employed for nucleic acid purification.

### DEFINITIONS

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen atom. Commonly, it is possible for the number of optional substituents, when present, to be 1, 2, 3, 4 or 5, in particular 1, 2 or 3.

As used herein, the term "one or more", e.g. in the definition of the substituents of the compounds of general formula (I) of the present invention, means "1, 2, 3, 4 or 5, particularly 1, 2, 3 or 4, more particularly 1, 2 or 3, even more particularly 1 or 2".

As used herein, an oxo substituent represents an oxygen atom, which is bound to a carbon atom or to a sulfur atom via a double bond.

The term "ring substituent" means a substituent attached to an aromatic or nonaromatic ring which replaces an available hydrogen atom on the ring.

The term "comprising" when used in the specification includes "consisting of".

If within the present text any item is referred to as "as mentioned herein", it means that it may be mentioned anywhere in the present text.

The terms as mentioned in the present text have the following meanings:
The term "halogen atom" means a fluorine, chlorine, bromine or iodine atom, particularly a fluorine, chlorine or bromine atom.

The term "C₁-C₆-alkyl" means a linear or branched, saturated, monovalent hydrocarbon group having 1, 2, 3, 4, 5 or 6 carbon atoms, e.g. a methyl, ethyl, propyl, isopropyl, butyl, sec-butyl, isobutyl, *tert-*butyl*,* pentyl, isopentyl, 2-methylbutyl, 1-methylbutyl, 1-ethylpropyl, 1,2-dimethylpropyl, *neo*-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer thereof. Particularly, said group has 1, 2, 3 or 4 carbon atoms ("C₁-C₄-alkyl"), *e.g*. a methyl, ethyl, propyl, isopropyl, butyl, sec-butyl, isobutyl, or *tert-*butyl group, more particularly 1, 2 or 3 carbon atoms ("C₁-C₃-alkyl"), *e.g*. a methyl, ethyl, *n*-propyl or isopropyl group.

The term "C₂-C₆-alkenyl" means a linear or branched, monovalent hydrocarbon group, which contains one or two double bonds, and which has 2, 3, 4, 5 or 6 carbon atoms, particularly 2 or 3 carbon atoms ("C₂-C₃-alkenyl"), it being understood that in the case in which said alkenyl group contains more than one double bond, then it is possible for said double bonds to be isolated from, or conjugated with, each other. Said alkenyl group is, for example, an ethenyl (or "vinyl"), prop-2-en-1-yl (or "allyl"), prop-1-en-1-yl, but-3-enyl, but-2-enyl, but-1-enyl, pent-4-enyl, pent-3-enyl, pent-2-enyl, pent-1-enyl, hex-5-enyl, hex-4-enyl, hex-3-enyl, hex-2-enyl, hex-1-enyl, prop-1-en-2-yl (or "isopropenyl"), 2-methylprop-2-enyl, 1-methylprop-2-enyl, 2-methylprop-1-enyl, 1-methylprop-1-enyl, 3-methylbut-3-enyl, 2-methylbut-3-enyl, 1-methylbut-3-enyl, 3-methylbut-2-enyl, 2-methylbut-2-enyl, 1-methylbut-2-enyl, 3-methylbut-1-enyl, 2-methylbut-1-enyl, 1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, 3-methylpent-3-enyl, 2-methylpent-3-enyl, 1-methylpent-3-enyl, 4-methylpent-2-enyl, 3-methylpent-2-enyl, 2-methylpent-2-enyl, 1-methylpent-2-enyl, 4-methylpent-1-enyl, 3-methylpent-1-enyl, 2-methylpent-1-enyl, 1-methylpent-1-enyl, 3-ethylbut-3-enyl, 2-ethylbut-3-enyl, 1-ethylbut-3-enyl, 3-ethylbut-2-enyl, 2-ethylbut-2-enyl, 1-ethylbut-2-enyl, 3-ethylbut-1-enyl, 2-ethylbut-1-enyl, 1-ethylbut-1-enyl, 2-propylprop-2-enyl, 1-propylprop-2-enyl, 2-isopropylprop-2-enyl, 1-isopropylprop-2-enyl, 2-propylprop-1-enyl, 1-propylprop-1-enyl, 2-isopropylprop-1-enyl, 1-isopropylprop-1-enyl, 3,3-dimethylprop-1-enyl, 1-(1,1-dimethylethyl)ethenyl, buta-1,3-dienyl, penta-1,4-dienyl or hexa-1,5-dienyl group. Particularly, said group is vinyl or allyl.

The term "spirocycloalkyl" means a saturated, monovalent bicyclic hydrocarbon group in which the two rings share one common ring carbon atom, and wherein said bicyclic hydrocarbon group contains 5, 6, 7, 8, 9, 10 or 11 carbon atoms, it being possible for said spirocycloalkyl group to be attached to the rest of the molecule via any one of the carbon atoms except the spiro carbon atom. Said spirocycloalkyl group is, for example, spiro[2.2]pentyl, spiro[2.3]hexyl, spiro[2.4]heptyl, spiro[2.5]octyl, spiro[2.6]nonyl, spiro[3.3]heptyl, spiro[3.4]octyl, spiro[3.5]nonyl, spiro[3.6]decyl, spiro[4.4]nonyl, spiro[4.5]decyl, spiro[4.6]undecyl or spiro[5.5]undecyl.

The terms "4- to 7-membered heterocycloalkyl" and "4- to 6-membered heterocycloalkyl" mean a monocyclic, saturated heterocycle with 4, 5, 6 or 7 or, respectively, 4, 5 or 6 ring atoms in total, which contains one or two identical or different ring heteroatoms from the series N, O and S, it being possible for said heterocycloalkyl group to be attached to the rest of the molecule via any one of the carbon atoms or, if present, a nitrogen atom.

Said heterocycloalkyl group, without being limited thereto, can be a 4-membered ring, such as azetidinyl, oxetanyl or thietanyl, for example; or a 5-membered ring, such as tetrahydrofuranyl, 1,3-dioxolanyl, thiolanyl, pyrrolidinyl, imidazolidinyl, pyrazolidinyl, 1,1-dioxidothiolanyl, 1,2-oxazolidinyl, 1,3-oxazolidinyl or 1,3-thiazolidinyl, for example; or a 6-membered ring, such as tetrahydropyranyl, tetrahydrothiopyranyl, piperidinyl, morpholinyl, dithianyl, thiomorpholinyl, piperazinyl, 1,3-dioxanyl, 1,4-dioxanyl or 1,2-oxazinanyl, for example, or a 7-membered ring, such as azepanyl, 1,4-diazepanyl or 1,4-oxazepanyl, for example.

Particularly, "4- to 6-membered heterocycloalkyl" means a 4- to 6-membered heterocycloalkyl as defined *supra* containing one ring nitrogen atom and optionally one further ring heteroatom from the series: N, O, S. More particularly, "5- or 6-membered heterocycloalkyl" means a monocyclic, saturated heterocycle with 5 or 6 ring atoms in total, containing one ring nitrogen atom and optionally one further ring heteroatom from the series: N, O.

The term "5- to 8-membered heterocycloalkenyl" means a monocyclic, unsaturated, nonaromatic heterocycle with 5, 6, 7 or 8 ring atoms in total, which contains one or two double bonds and one or two identical or different ring heteroatoms from the series: N, O, S; it being possible for said heterocycloalkenyl group to be attached to the rest of the molecule via any one of the carbon atoms or, if present, a nitrogen atom.

Said heterocycloalkenyl group is, for example, 4*H*-pyranyl, 2*H*-pyranyl, 2,5-dihydro-1*H*-pyrrolyl, [1,3]dioxolyl, 4*H*-[1,3,4]thiadiazinyl, 2,5-dihydrofuranyl, 2,3-dihydrofuranyl, 2,5-dihydrothiophenyl, 2,3-dihydrothiophenyl, 4,5-dihydrooxazolyl or 4*H*-[1,4]thiazinyl.

The term "heterospirocycloalkyl" means a bicyclic, saturated heterocycle with 6, 7, 8, 9, 10 or 11 ring atoms in total, in which the two rings share one common ring carbon atom, which "heterospirocycloalkyl" contains one or two identical or different ring heteroatoms from the series: N, O, S; it being possible for said heterospirocycloalkyl group to be attached to the rest of the molecule via any one of the carbon atoms, except the spiro carbon atom, or, if present, a nitrogen atom.

Said heterospirocycloalkyl group is, for example, azaspiro[2.3]hexyl, azaspiro[3.3]heptyl, oxaazaspiro[3.3]heptyl, thiaazaspiro[3.3]heptyl, oxaspiro[3.3]heptyl, oxazaspiro[5.3]nonyl, oxazaspiro[4.3]octyl, azaspiro[4,5]decyl, oxazaspiro [5.5]undecyl, diazaspiro[3.3]heptyl, thiazaspiro[3.3]heptyl, thiazaspiro[4.3]octyl, azaspiro[5.5]undecyl, or one of the further homologous scaffolds such as spiro[3.4]-, spiro[4.4]-, spiro[2.4]-, spiro[2.5]-, spiro[2.6]-, spiro[3.5]-, spiro[3.6]-, spiro[4.5]- and spiro[4.6]-.

The term "fused heterocycloalkyl" means a bicyclic, saturated heterocycle with 6, 7, 8, 9 or 10 ring atoms in total, in which the two rings share two adjacent ring atoms, which "fused heterocycloalkyl" contains one or two identical or different ring heteroatoms from the series: N, O, S; it being possible for said fused heterocycloalkyl group to be attached to the rest of the molecule via any one of the carbon atoms or, if present, a nitrogen atom.

Said fused heterocycloalkyl group is, for example, azabicyclo[3.3.0]octyl, azabicyclo[4.3.0]nonyl, diazabicyclo[4.3.0]nonyl, oxazabicyclo[4.3.0]nonyl, thiazabicyclo[4.3.0]nonyl or azabicyclo[4.4.0]decyl.

The term "bridged heterocycloalkyl" means a bicyclic, saturated heterocycle with 7, 8, 9 or 10 ring atoms in total, in which the two rings share two common ring atoms which are not adjacent, which "bridged heterocycloalkyl" contains one or two identical or different ring heteroatoms from the series: N, O, S; it being possible for said bridged heterocycloalkyl group to be attached to the rest of the molecule via any one of the carbon atoms, except the spiro carbon atom, or, if present, a nitrogen atom.

Said bridged heterocycloalkyl group is, for example, azabicyclo[2.2.1]heptyl, oxazabicyclo[2.2.1]heptyl, thiazabicyclo[2.2.1]heptyl, diazabicyclo[2.2.1]heptyl, azabicyclo-[2.2.2]octyl, diazabicyclo[2.2.2]octyl, oxazabicyclo[2.2.2]octyl, thiazabicyclo[2.2.2]octyl, azabicyclo[3.2.1]octyl, diazabicyclo[3.2.1]octyl, oxazabicyclo[3.2.1]octyl, thiazabicyclo[3.2.1]octyl, azabicyclo[3.3.1]nonyl, diazabicyclo[3.3.1]nonyl, oxazabicyclo[3.3.1]nonyl, thiazabicyclo[3.3.1]-nonyl, azabicyclo[4.2.1]nonyl, diazabicyclo[4.2.1]nonyl, oxazabicyclo[4.2.1]nonyl, thiazabicyclo[4.2.1]nonyl, azabicyclo[3.3.2]decyl, diazabicyclo[3.3.2]decyl, oxazabicyclo[3.3.2]decyl, thiazabicyclo[3.3.2]decyl or azabicyclo[4.2.2]decyl.

The term "heteroaryl" means a monovalent, monocyclic, bicyclic or tricyclic aromatic ring having 5, 6, 8, 9, 10, 11, 12, 13 or 14 ring atoms (a "5- to 14-membered heteroaryl" group), particularly 5, 6, 9 or 10 ring atoms, which contains at least one ring heteroatom and optionally one, two or three further ring heteroatoms from the series: N, O and/or S, and which is bound via a ring carbon atom or optionally via a ring nitrogen atom (if allowed by valency).

Said heteroaryl group can be a 5-membered heteroaryl group, such as, for example, thienyl, furanyl, pyrrolyl, oxazolyl, thiazolyl, imidazolyl, pyrazolyl, isoxazolyl, isothiazolyl, oxadiazolyl, triazolyl, thiadiazolyl or tetrazolyl; or a 6-membered heteroaryl group, such as, for example, pyridinyl, pyridazinyl, pyrimidinyl, pyrazinyl or triazinyl; or a tricyclic heteroaryl group, such as, for example, carbazolyl, acridinyl or phenazinyl; or a 9-membered heteroaryl group, such as, for example, benzofuranyl, benzothienyl, benzoxazolyl, benzisoxazolyl, benzimidazolyl, benzothiazolyl, benzotriazolyl, indazolyl, indolyl, isoindolyl, indolizinyl or purinyl; or a 10-membered heteroaryl group, such as, for example, quinolinyl, quinazolinyl, isoquinolinyl, cinnolinyl, phthalazinyl, quinoxalinyl or pteridinyl.

In general, and unless otherwise mentioned, the heteroaryl or heteroarylene groups include all possible isomeric forms thereof, e.g.: tautomers and positional isomers with respect to the point of linkage to the rest of the molecule. Thus, for some illustrative non-restricting examples, the term pyridinyl includes pyridin-2-yl, pyridin-3-yl and pyridin-4-yl; or the term thienyl includes thien-2-yl and thien-3-yl.

The term "C₁-C₆", as used in the present text, e.g. in the context of the definition of "C₁-C₆-alkyl", "C₁-C₆-haloalkyl", "C₁-C₆-hydroxyalkyl", "C₁-C₆-alkoxy" or "C₁-C₆-haloalkoxy" means an alkyl group having a finite number of carbon atoms of 1 to 6, *i.e.* 1, 2, 3, 4, 5 or 6 carbon atoms.

Further, as used herein, the term "C₃-C₈", as used in the present text, *e.g.* in the context of the definition of "C₃-C₈-cycloalkyl", means a cycloalkyl group having a finite number of carbon atoms of 3 to 8, *i.e.* 3, 4, 5, 6, 7 or 8 carbon atoms.

When a range of values is given, said range encompasses each value and sub-range within said range.

For example:
"C₁-C₆" encompasses C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, and C₅-C₆;
"C₂-C₆" encompasses C₂, C₃, C₄, C₅, C₆, C₂-C₆, C₂-C₅, C₂-C₄, C₂-C₃, C₃-C₆, C₃-C₅, C₃-C₄, C₄-C₆, C₄-C₅, and C₅-C₆;
"C₃-C₁₀" encompasses C₃, C₄, C₅, C₆, C₇, C₈, C₉, C₁₀, C₃-C₁₀, C₃-C₉, C₃-C₈, C₃-C₇, C₃-C₆, C₃-C₅, C₃-C₄, C₄-C₁₀, C₄-C₉, C₄-C₈, C₄-C₇, C₄-C₆, C₄-C₅, C₅-C₁₀, C₅-C₉, C₅-C₈, C₅-C₇, C₅-C₆, C₆-C₁₀, C₆-C₉, C₆-C₈, C₆-C₇, C₇-C₁₀, C₇-C₉, C₇-C₈, C₈-C₁₀, C₈-C₉ and C₉-C₁₀;
"C₃-C₈" encompasses C₃, C₄, C₅, C₆, C₇, C₈, C₃-C₈, C₃-C₇, C₃-C₆, C₃-C₅, C₃-C₄, C₄-C₈, C₄-C₇, C₄-C₆, C₄-C₅, C₅-C₈, C₅-C₇, C₅-C₆, C₆-C₈, C₆-C₇ and C₇-C₈;
"C₃-C₆" encompasses C₃, C₄, C₅, C₆, C₃-C₆, C₃-C₅, C₃-C₄, C₄-C₆, C₄-C₅, and C₅-C₆;
"C₄-C₈" encompasses C₄, C₅, C₆, C₇, C₈, C₄-C₈, C₄-C₇, C₄-C₆, C₄-C₅, C₅-C₈, C₅-C₇, C₅-C₆, C₆-C₈, C₆-C₇ and C₇-C₈;
"C₄-C₇" encompasses C₄, C₅, C₆, C₇, C₄-C₇, C₄-C₆, C₄-C₅, C₅-C₇, C₅-C₆ and C₆-C₇;
"C₄-C₆" encompasses C₄, C₅, C₆, C₄-C₆, C₄-C₅ and C₅-C₆;
"C_{S}-C₁₀" encompasses C₅, C₆, C₇, C₈, C₉, C₁₀, C₈-C₁₀, C₅-C₉, C₅-C₈, C₅-C₇, C₅-C₆, C₆-C₁₀, C₆-C₉, C₆-C₈, C₆-C₇, C₇-C₁₀, C₇-C₉, C₇-C₈, C₈-C₁₀, C₈-C₉ and C₉-C₁₀;
"C₆-C₁₀" encompasses C₆, C₇, C₈, C₉, C₁₀, C₆-C₁₀, C₆-C₉, C₆-C₈, C₆-C₇, C₇-C₁₀, C₇-C₉, C₇-C₈, C₈-C₁₀, C₈-C₉ and C₉-C₁₀.

As used herein, the term "leaving group" means an atom or a group of atoms that is displaced in a chemical reaction as stable species taking with it the bonding electrons. In particular, such a leaving group is selected from the group comprising: halide, in particular fluoride, chloride, bromide or iodide, (methylsulfonyl)oxy, [(trifluoromethyl)sulfonyl]oxy, [(nonafluorobutyl)-sulfonyl]oxy, (phenylsulfonyl)oxy, [(4-methylphenyl)sulfonyl]oxy, [(4-bromophenyl)sulfonyl]oxy, [(4-nitrophenyl)sulfonyl]oxy, [(2-nitrophenyl)sulfonyl]oxy, [(4-isopropylphenyl)sulfonyl]oxy, [(2,4,6-triisopropylphenyl)sulfonyl]oxy, [(2,4,6-trimethylphenyl)sulfonyl]oxy, *[(4-tert-butyl*phenyl)sulfonyl]oxy and [(4-methoxyphenyl)sulfonyl]oxy.

Administration: The term "administration" typically refers to the delivery of a composition to a subject or system. Various routes may be utilized for administration to a subject, such as a human, depending on the circumstances. Examples of administration routes include ocular, oral, parenteral, and topical. Specific routes may include bronchial (e.g., by bronchial instillation), buccal, dermal (e.g., topical to the dermis, intradermal, interdermal, transdermal), enteral, intraarterial, intradermal, intragastric, intramedullary, intramuscular, intranasal, intraperitoneal, intrathecal, intravenous, intraventricular, within a specific organ (e.g., intrahepatic), mucosal, nasal, oral, rectal, subcutaneous, sublingual, topical, tracheal (e.g., by intratracheal instillation), vaginal, vitreal, etc. Administration can involve intermittent dosing (e.g., multiple doses separated in time) and/or periodic dosing (e.g., doses separated by a common period of time).

Continuous dosing (e.g., perfusion) for a selected period of time is also possible. A pharmaceutical composition comprising lipid nanoparticles can be formulated for administration via parenteral (intramuscular, intraperitoneal, intravenous (IV) or subcutaneous injection), transdermal (either passively or using iontophoresis or electroporation), or transmucosal (nasal, vaginal, rectal, or sublingual) routes, or using bioerodible inserts. Dosage forms can be tailored for each route of administration.

An amino acid, in its broadest sense as used herein, refers to any compound or substance capable of being incorporated into a polypeptide chain through the formation of one or more peptide bonds. In certain embodiments, an amino acid possesses the general structure H2N-C(H)(R)-COOH. Amino acids can be naturally occurring or non-natural; they may also be classified as D-amino acids or L-amino acids. The term "standard amino acid" pertains to the twenty common L-amino acids frequently found in natural peptides, whereas "nonstandard amino acid" refers to any amino acid other than the standard ones, irrespective of whether it is synthesized or sourced naturally.

Amino acids, including those located at carboxyl- and/or amino-terminal positions in a polypeptide, may exhibit structural modifications relative to the aforementioned general structure. Modifications can include methylation, amidation, acetylation, pegylation, glycosylation, phosphorylation, or substitution of functional groups such as amino, carboxylic acid, protons, or hydroxyl groups. These modifications can affect the circulating half-life of a polypeptide containing the modified amino acid compared to one with an unmodified version. However, such modifications might not significantly impact the relevant activity of the polypeptide. Depending on the context, "amino acid" may refer to a free amino acid or an amino acid residue within a polypeptide.

Animal: As used herein refers to any member of the animal kingdom. In some embodiments, "animal" refers to humans, of either sex and at any stage of development. In some embodiments, "animal" refers to non-human animals, at any stage of development. In certain embodiments, the non-human animal is a mammal (e.g., a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, insects, and/or worms. In some embodiments, an animal may be a transgenic animal, genetically engineered animal, and/or a clone.

Aptamer: As used herein, the term "aptamer" refers to a macromolecule composed of nucleic acid (e.g., RNA, DNA) that binds specifically to a molecular target (e.g., an umbrella topology glycan). A particular aptamer may be described by a linear nucleotide sequence and is typically about 15-60 nucleotides in length. It is contemplated that the chain of nucleotides in an aptamer forms intramolecular interactions that fold the molecule into a complex three-dimensional shape, allowing the aptamer to bind closely to its target molecule. Given the diversity of molecular shapes within the universe of nucleotide sequences, aptamers may be obtained for a wide array of molecular targets, including proteins and small molecules. Aptamers usually have high specificity and affinities for their targets (e.g., affinities in the picomolar to low nanomolar range for proteins). In many embodiments, aptamers are chemically stable and can endure boiling or freezing without losing activity. As synthetic molecules, aptamers can be modified to enhance their function for specific applications. For example, aptamers can be altered to reduce sensitivity to enzymatic degradation in the blood for in vivo use or to change their biodistribution or plasma residence time.

Associated: Two events or entities are considered "associated" if the presence, level, degree, type, and/or form of one correlates with that of the other. For example, a specific entity (such as a polypeptide, genetic signature, metabolite, microbe, etc.) is regarded as associated with a particular disease, disorder, or condition if its presence, level, and/or form correlates with incidence of and/or susceptibility to the disease, disorder, or condition across a relevant population. In some instances, two or more entities are physically "associated" if they interact directly or indirectly, thereby remaining in physical proximity. This association can be covalent or non-covalent, including interactions like hydrogen bonds, van der Waals interactions, hydrophobic interactions, magnetism, and combinations thereof.

Biocompatible: The term "biocompatible" refers to materials that do not cause significant harm to living tissue when placed in contact with such tissue, e.g., in vivo. In certain embodiments, materials are biocompatible if they are not toxic to cells. Further, materials are biocompatible if their addition to cells in vitro results in less than or equal to 20% cell death, and/or their administration in vivo does not induce significant inflammation or other adverse effects.

Biodegradable: "Biodegradable" pertains to materials that, when introduced into cells, are broken down by cellular machinery (such as enzymatic degradation, hydrolysis, or combinations thereof) into components that cells can reuse or dispose of without significant toxic effects. Components generated from biodegradable materials should be biocompatible, not inducing significant inflammation or adverse effects in vivo. Biodegradable polymer materials may break down into their component monomers or involve hydrolysis of ester bonds or cleavage of urethane linkages. Examples include polymers of hydroxy acids like lactic acid and glycolic acid, poly(hydroxy acids), PLA, PGA, PLGA, copolymers with PEG, polyanhydrides, poly(ortho)esters, polyesters, polyurethanes, among others. Naturally occurring polymers, such as proteins like albumin, collagen, gelatin, and prolamines (e.g., zein), as well as polysaccharides like alginate, cellulose derivatives, and polyhydroxyalkanoates (e.g., polyhydroxybutyrate blends and copolymers), also exhibit biodegradability. Professionals in the field will recognize or determine the biocompatibility and/or biodegradability of these polymers and their derivatives.

Biologically active: as used herein, refers to an observable biological effect or result achieved by an agent or entity of interest. For example, in some embodiments, a specific binding interaction is a biological activity. In some embodiments, modulation (e.g., induction, enhancement, or inhibition) of a biological pathway or event is a biological activity. In some embodiments, presence or extent of a biological activity is assessed through detection of a direct or indirect product produced by a biological pathway or event of interest.

Biological sample: As used herein, the term "biological sample" typically refers to a sample obtained or derived from a biological source (e.g., a tissue or organism or cell culture) of interest, as described herein. In some embodiments, a source of interest comprises an organism, such as an animal or human. In some embodiments, a biological sample is or comprises biological tissue or fluid. In some embodiments, a biological sample may be or comprise bone marrow; blood; blood cells; ascites; tissue or fine needle biopsy samples; cell-containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or broncheoalveolar lavages; aspirates; scrapings; bone marrow specimens; tissue biopsy specimens; surgical specimens; feces, other body fluids, secretions, and/or excretions; and/or cells therefrom, etc. In some embodiments, a biological sample is or comprises cells obtained from an individual. In some embodiments, obtained cells are or include cells from an individual from whom the sample is obtained. In some embodiments, a sample is a "primary sample" obtained directly from a source of interest by any appropriate means. For example, in some embodiments, a primary biological sample is obtained by methods selected from the group consisting of biopsy (e.g., fine needle aspiration or tissue biopsy), surgery, collection of body fluid (e.g., blood, lymph, feces etc.), etc. In some embodiments, as will be clear from context, the term "sample" refers to a preparation that is obtained by processing (e.g., by removing one or more components of and/or by adding one or more agents to) a primary sample. For example, filtering using a semi-permeable membrane. Such a "processed sample" may comprise, for example nucleic acids or proteins extracted from a sample or obtained by subjecting a primary sample to techniques such as amplification or reverse transcription of mRNA, isolation and/or purification of certain components, etc.

Cancer: The terms "cancer", "malignancy", "neoplasm", "tumor", and "carcinoma", are used herein to refer to cells that exhibit relatively abnormal, uncontrolled, and/or autonomous growth, so that they exhibit an aberrant growth phenotype characterized by a significant loss of control of cell proliferation. In some embodiments, a tumor may be or comprise cells that are precancerous (e.g., benign), malignant, pre-metastatic, metastatic, and/or non-metastatic. The present disclosure specifically identifies certain cancers to which its teachings may be particularly relevant. In some embodiments, a relevant cancer may be characterized by a solid tumor. In some embodiments, a relevant cancer may be characterized by a hematologic tumor. In general, examples of different types of cancers known in the art include, for example, hematopoietic cancers including leukemias, lymphomas (Hodgkin's and non-Hodgkin's), myelomas and myeloproliferative disorders; sarcomas, melanomas, adenomas, carcinomas of solid tissue, squamous cell carcinomas of the mouth, throat, larynx, and lung, liver cancer, genitourinary cancers such as prostate, cervical, bladder, uterine, and endometrial cancer and renal cell carcinomas, bone cancer, pancreatic cancer, skin cancer, cutaneous or intraocular melanoma, cancer of the endocrine system, cancer of the thyroid gland, cancer of the parathyroid gland, head and neck cancers, breast cancer, gastro-intestinal cancers and nervous system cancers, benign lesions such as papillomas, and the like.

Carrier: as used herein, refers to a diluent, adjuvant, excipient, or vehicle with which a composition is administered. In some exemplary embodiments, carriers can include sterile liquids, such as, for example, water and oils, including oils of petroleum, animal, vegetable or synthetic origin, such as, for example, peanut oil, soybean oil, mineral oil, sesame oil and the like. In some embodiments, carriers are or include one or more solid components. Comparable: As used herein, the term "comparable" refers to two or more agents, entities, situations, sets of conditions, etc., that may not be identical to one another but that are sufficiently similar to permit comparison therebetween so that one skilled in the art will appreciate that conclusions may reasonably be drawn based on differences or similarities observed. In some embodiments, comparable sets of conditions, circumstances, individuals, or populations are characterized by a plurality of substantially identical features and one or a small number of varied features. Those of ordinary skill in the art will understand, in context, what degree of identity is required in any given circumstance for two or more such agents, entities, situations, sets of conditions, etc., to be considered comparable. For example, those of ordinary skill in the art will appreciate that sets of circumstances, individuals, or populations are comparable to one another when characterized by a sufficient number and type of substantially identical features to warrant a reasonable conclusion that differences in results obtained or phenomena observed under or with different sets of circumstances, individuals, or populations are caused by or indicative of the variation in those features that are varied.

Composition: Those skilled in the art will appreciate that the term "composition" may be used to refer to a discrete physical entity that comprises one or more specified components. In general, unless otherwise specified, a composition may be of any form - e.g., gas, gel, liquid, solid, etc.

"Improve," "increase", "inhibit" or "reduce": As used herein, the terms "improve", "increase", "inhibit", "reduce", or grammatical equivalents thereof, indicate values that are relative to a baseline or other reference measurement. In some embodiments, an appropriate reference measurement may be or comprise a measurement in a particular system (e.g., in a single individual) under otherwise comparable conditions absent presence of (e.g., prior to and/or after) a particular agent or treatment, or in presence of an appropriate comparable reference agent. In some embodiments, an appropriate reference measurement may be or comprise a measurement in a comparable system known or expected to respond in a particular way, in presence of the relevant agent or treatment.

Determine: Many methodologies described herein include a step of "determining". Those of ordinary skill in the art, reading the present specification, will appreciate that such "determining" can utilize or be accomplished through use of any of a variety of techniques available to those skilled in the art, including for example specific techniques explicitly referred to herein. In some embodiments, determining involves manipulation of a physical sample. In some embodiments, determining involves consideration and/or manipulation of data or information, for example utilizing a computer or other processing unit adapted to perform a relevant analysis. In some embodiments, determining involves receiving relevant information and/or materials from a source. In some embodiments, determining involves comparing one or more features of a sample or entity to a comparable reference.

Encapsulated: The term "encapsulated" is used herein to refer to substances that are completely surrounded by another material.

Excipient: as used herein, refers to a non-therapeutic agent that may be included in a pharmaceutical composition, for example to provide or contribute to a desired consistency or stabilizing effect. Suitable pharmaceutical excipients include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

Expression: As used herein, the term "expression" of a nucleic acid sequence refers to the generation of any gene product from the nucleic acid sequence. In some embodiments, a gene product can be a transcript. In some embodiments, a gene product can be a polypeptide. In some embodiments, expression of a nucleic acid sequence involves one or more of the following: (1) production of an RNA template from a DNA sequence (e.g., by transcription); (2) processing of an RNA transcript (e.g., by splicing, editing, 5' cap formation, and/or 3' end formation); (3) translation of an RNA into a polypeptide or protein; and/or (4) post-translational modification of a polypeptide or protein.

Inhibitory agent: As used herein, the term "inhibitory agent" refers to an entity, condition, or event whose presence, level, or degree correlates with decreased level or activity of a target). In some embodiments, an inhibitory agent may act directly (in which case it exerts its influence directly upon its target, for example by binding to the target); in some embodiments, an inhibitory agent may act indirectly (in which case it exerts its influence by interacting with and/or otherwise altering a regulator of the target, so that level and/or activity of the target is reduced). In some embodiments, an inhibitory agent is one whose presence or level correlates with a target level or activity that is reduced relative to a particular reference level or activity (e.g., that observed under appropriate reference conditions, such as presence of a known inhibitory agent, or absence of the inhibitory agent in question, etc).

In vitro: The term "in vitro" as used herein refers to events that occur in an artificial environment, e.g., in a test tube or reaction vessel, in cell culture, etc., rather than within a multi-cellular organism.

In vivo: as used herein refers to events that occur within a multi-cellular organism, such as a human and a non-human animal. In the context of cell-based systems, the term may be used to refer to events that occur within a living cell (as opposed to, for example, in vitro systems).

Linker: as used herein, is used to refer to that portion of a multi-element agent that connects different elements to one another. For example, those of ordinary skill in the art appreciate that a polypeptide whose structure includes two or more functional or organizational domains often includes a stretch of amino acids between such domains that links them to one another. In some embodiments, a polypeptide comprising a linker element "L'" has an overall structure of the general form S1-L'-S2, wherein S1 and S2 may be the same or different and represent two domains associated with one another by the linker. In some embodiments, a polyptide linker is at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100 or more amino acids in length. In some embodiments, a linker is characterized in that it tends not to adopt a rigid three-dimensional structure, but rather provides flexibility to the polypeptide. A variety of different linker elements that can appropriately be used when engineering polypeptides (e.g., fusion polypeptides) known in the art (see e.g., Holliger, P., et al. (1993) Proc. Natl. Acad. Sci. USA 90:6444-6448; Poljak, R. J., et al. (1994) Structure 2: 1121-1123).

Improve, increase, inhibit or reduce: As used herein, the terms "improve", "increase", "inhibit", "reduce", or grammatical equivalents thereof, indicate values that are relative to a baseline or other reference measurement. In some embodiments, an appropriate reference measurement may be or comprise a measurement in a particular system (e.g., in a single individual) under otherwise comparable conditions absent presence of (e.g., prior to and/or after) a particular agent or treatment, or in presence of an appropriate comparable reference agent. In some embodiments, an appropriate reference measurement may be or comprise a measurement in a comparable system known or expected to respond in a particular way, in presence of the relevant agent or treatment.

Determine: Many methodologies described herein include a step of "determining". Those of ordinary skill in the art, reading the present specification, will appreciate that such "determining" can utilize or be accomplished through use of any of a variety of techniques available to those skilled in the art, including for example specific techniques explicitly referred to herein. In some embodiments, determining involves manipulation of a physical sample. In some embodiments, determining involves consideration and/or manipulation of data or information, for example utilizing a computer or other processing unit adapted to perform a relevant analysis. In some embodiments, determining involves receiving relevant information and/or materials from a source. In some embodiments, determining involves comparing one or more features of a sample or entity to a comparable reference.

Encapsulated: The term "encapsulated" is used herein to refer to substances that are completely surrounded by another material.

Excipient: as used herein, refers to a non-therapeutic agent that may be included in a pharmaceutical composition, for example to provide or contribute to a desired consistency or stabilizing effect. Suitable pharmaceutical excipients include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

Expression: As used herein, the term "expression" of a nucleic acid sequence refers to the generation of any gene product from the nucleic acid sequence. In some embodiments, a gene product can be a transcript. In some embodiments, a gene product can be a polypeptide. In some embodiments, expression of a nucleic acid sequence involves one or more of the following: (1) production of an RNA template from a DNA sequence (e.g., by transcription); (2) processing of an RNA transcript (e.g., by splicing, editing, 5' cap formation, and/or 3' end formation); (3) translation of an RNA into a polypeptide or protein; and/or (4) post-translational modification of a polypeptide or protein.

Inhibitory agent: As used herein, the term "inhibitory agent" refers to an entity, condition, or event whose presence, level, or degree correlates with decreased level or activity of a target). In some embodiments, an inhibitory agent may act directly (in which case it exerts its influence directly upon its target, for example by binding to the target); in some embodiments, an inhibitory agent may act indirectly (in which case it exerts its influence by interacting with and/or otherwise altering a regulator of the target, so that level and/or activity of the target is reduced). In some embodiments, an inhibitory agent is one whose presence or level correlates with a target level or activity that is reduced relative to a particular reference level or activity (e.g., that observed under appropriate reference conditions, such as presence of a known inhibitory agent, or absence of the inhibitory agent in question, etc).

In vitro: The term "in vitro" as used herein refers to events that occur in an artificial environment, e.g., in a test tube or reaction vessel, in cell culture, etc., rather than within a multi-cellular organism.

In vivo: as used herein refers to events that occur within a multi-cellular organism, such as a human and a non-human animal. In the context of cell-based systems, the term may be used to refer to events that occur within a living cell (as opposed to, for example, in vitro systems).

Linker: as used herein, is used to refer to that portion of a multi-element agent that connects different elements to one another. For example, those of ordinary skill in the art appreciate that a polypeptide whose structure includes two or more functional or organizational domains often includes a stretch of amino acids between such domains that links them to one another. In some embodiments, a polypeptide comprising a linker element "L'" has an overall structure of the general form S1-L'-S2, wherein S1 and S2 may be the same or different and represent two domains associated with one another by the linker. In some embodiments, a polyptide linker is at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100 or more amino acids in length. In some embodiments, a linker is characterized in that it tends not to adopt a rigid three-dimensional structure, but rather provides flexibility to the polypeptide. A variety of different linker elements that can appropriately be used when engineering polypeptides (e.g., fusion polypeptides) known in the art (see e.g., Holliger, P., et al. (1993) Proc. Natl. Acad. Sci. USA 90:6444-6448; Poljak, R. J., et al. (1994) Structure 2: 1121-1123).

Nanoparticle: The term "nanoparticle" as used herein refers to a particle having a diameter of less than 1000 nanometers (nm). In some instances, a nanoparticle has a diameter of less than 300 nm, as defined by the National Science Foundation. In other cases, a nanoparticle has a diameter of less than 100 nm, as defined by the National Institutes of Health. Nanoparticles can be micelles, consisting of an enclosed compartment separated from the bulk solution by a micellar membrane typically composed of amphiphilic entities that surround and enclose a space or compartment. A micellar membrane may consist of at least one polymer, such as a biocompatible and/or biodegradable polymer. Lipid nanoparticles described here can have an average hydrodynamic diameter ranging from about 30 to about 170 nm. They can also have specific hydrodynamic diameters, including values like 30 nm, 35 nm, 40 nm, up to 170 nm, or any range between these values. For example, lipid nanoparticles can have an average hydrodynamic diameter between 50 nm and 100 nm.

Nanoparticle composition: As used herein, the term "nanoparticle composition" refers to a composition that contains at least one nanoparticle and at least one additional agent or ingredient. In some embodiments, a nanoparticle composition contains a substantially uniform collection of nanoparticles as described herein.

Nucleic acid: As used herein, in its broadest sense, refers to any compound and/or substance that is or can be incorporated into an oligonucleotide chain. In some embodiments, a nucleic acid is a compound and/or substance that is or can be incorporated into an oligonucleotide chain via a phosphodiester linkage. As will be clear from context, in some embodiments, "nucleic acid" refers to an individual nucleic acid residue (e.g., a nucleotide and/or nucleoside); in some embodiments, "nucleic acid" refers to an oligonucleotide chain comprising individual nucleic acid residues. In some embodiments, a "nucleic acid" is or comprises RNA; in some embodiments, a "nucleic acid" is or comprises DNA. In some embodiments, a nucleic acid is, comprises, or consists of one or more natural nucleic acid residues. In some embodiments, a nucleic acid is, comprises, or consists of one or more nucleic acid analogs. In some embodiments, a nucleic acid analog differs from a nucleic acid in that it does not utilize a phosphodiester backbone. For example, in some embodiments, a nucleic acid is, comprises, or consists of one or more "peptide nucleic acids", which are known in the art and have peptide bonds instead of phosphodiester bonds in the backbone, are considered within the scope of the present invention. Alternatively or additionally, in some embodiments, a nucleic acid has one or more phosphorothioate and/or 5'-N- phosphoramidite linkages rather than phosphodiester bonds. In some embodiments, a nucleic acid is, comprises, or consists of one or more natural nucleosides (e.g., adenosine, thymidine, guanosine, cytidine, uridine, deoxyadenosine, deoxythymidine, deoxy guanosine, and deoxycytidine). In some embodiments, a nucleic acid is, comprises, or consists of one or more nucleoside analogs (e.g., 2-aminoadenosine, 2-thiothymidine, inosine, pyrrolo-pyrimidine, 3 - methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5 - propynyl-cytidine, C5-methylcytidine, 2-aminoadenosine, 7-deazaadenosine, 7-deazaguanosine, 8-oxoadenosine, 8-oxoguanosine, O(6)-methylguanine, 2-thiocytidine, methylated bases, intercalated bases, and combinations thereof). In some embodiments, a nucleic acid comprises one or more modified sugars (e.g., 2'-fluororibose, ribose, 2'-deoxyribose, arabinose, and hexose) as compared with those in natural nucleic acids. In some embodiments, a nucleic acid has a nucleotide sequence that encodes a functional gene product such as an RNA or protein. In some embodiments, a nucleic acid includes one or more introns. In some embodiments, nucleic acids are prepared by one or more of isolation from a natural source, enzymatic synthesis by polymerization based on a complementary template (in vivo or in vitro), reproduction in arecombinant cell or system, and chemical synthesis. In some embodiments, a nucleic acid is at least 3, 4, 5, 6, 7, 8, 9, 10, 15, 20, 25, 30, 35, 40, 45, 50, 55, 60, 65, 70, 75, 80, 85, 90, 95, 100, 1 10, 120, 130, 140, 150, 160, 170, 180, 190, 20, 225, 250, 275, 300, 325, 350, 375, 400, 425, 450, 475, 500, 600, 700, 800, 900, 1000, 1500, 2000, 2500, 3000, 3500, 4000, 4500, 5000 or more residues long. In some embodiments, a nucleic acid is partly or wholly single stranded; in some embodiments, a nucleic acid is partly or wholly double stranded. In some embodiments a nucleic acid has a nucleotide sequence comprising at least one element that encodes, or is the complement of a sequence that encodes, a polypeptide. In some embodiments, a nucleic acid has enzymatic activity.

Operably linked: as used herein, refers to the arrangement of components such that they can function together as intended. When a control element is "operably linked" to a functional element, it means that the control element facilitates the expression and/or activity of the functional element under compatible conditions. In some embodiments, control elements may be directly adjacent (e.g., covalently linked) to the coding sequences of interest; in other cases, they may act from a distance or in trans relative to the functional element.

For purposes of this invention, chemical elements are identified in accordance with the Periodic Table of the Elements, CAS version, as found in the Handbook of Chemistry and Physics, 75th Edition. Additionally, general principles of organic chemistry are described in "Organic Chemistry" by Thomas Sorrell, and "March's Advanced Organic Chemistry", 5th Edition by M.B. Smith and J. March, the entirety of which are hereby incorporated by reference.

Parenteral: The phrases "parenteral administration" and "administered parenterally" as used herein refer to methods of delivery other than oral and topical, typically involving injections. These include, but are not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, and intrasternal injection and infusion.

Patient: As used herein, the term "patient" refers to any organism that receives a given composition for purposes such as experimental, diagnostic, prophylactic, cosmetic, and/or therapeutic treatment. Common examples include animals (e.g., mammals like mice, rats, rabbits, non-human primates, and/or humans). In some instances, the patient is a human. In some instances, the patient is suffering from or at risk of one or more disorders or conditions. In some instances, the patient exhibits one or more symptoms of a disorder or condition. In some instances, the patient has been diagnosed with one or more disorders or conditions. In some instances, the disorder or condition involves cancer or the presence of one or more tumors. In some instances, the patient is undergoing or has undergone certain therapies to diagnose and/or treat a disease, disorder, or condition.

Pharmaceutical composition: As used herein, the term "pharmaceutical composition" refers to an active agent formulated with one or more pharmaceutically acceptable carriers. In some instances, the active agent is present in a unit dose appropriate for administration in a therapeutic regimen, showing a statistically significant probability of achieving a predetermined therapeutic effect when administered to a relevant population. In some instances, pharmaceutical compositions may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous, or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream, or foam; sublingually; ocularly; transdermally; or nasally, pulmonary, and to other mucosal surfaces.

Pharmaceutically acceptable: As used herein, the phrase "pharmaceutically acceptable" pertains to those compounds, materials, compositions, and/or dosage forms which are suitable for use in contact with the tissues of humans and animals without causing excessive toxicity, irritation, allergic response, or other problems, ensuring a reasonable benefit/risk ratio.

Pharmaceutically acceptable carrier: As used herein, the term "pharmaceutically acceptable carrier" refers to a pharmaceutically-acceptable material, composition, or vehicle, such as a liquid or solid filler, diluent, excipient, or solvent encapsulating material, involved in carrying or transporting the subject compound from one organ or part of the body to another. Each carrier must be "acceptable" in the sense of being compatible with the other ingredients of the formulation and not injurious to the patient. Examples of pharmaceutically-acceptable carriers include: sugars (like lactose, glucose, and sucrose); starches (such as corn starch and potato starch); cellulose and its derivatives (including sodium carboxymethyl cellulose, ethyl cellulose, and cellulose acetate); powdered tragacanth; malt; gelatin; talc; excipients such as cocoa butter and suppository waxes; oils (e.g., peanut oil, cottonseed oil, safflower oil, sesame oil, olive oil, corn oil, and soybean oil); glycols (such as propylene glycol); polyols (including glycerin, sorbitol, mannitol, and polyethylene glycol); esters (like ethyl oleate and ethyl laurate); agar; buffering agents (e.g., magnesium hydroxide and aluminum hydroxide); alginic acid; pyrogen-free water; isotonic saline; Ringer's solution; ethyl alcohol; pH buffered solutions; polyesters, polycarbonates, and/or polyanhydrides; and other non-toxic compatible substances employed in pharmaceutical formulations.

Pharmaceutically acceptable salt: The term "pharmaceutically acceptable salt," as used herein, refers to salts of such compounds that are suitable for use in pharmaceutical contexts, meaning salts which are appropriate for use in contact with the tissues of humans and lower animals without undue toxicity, irritation, allergic response, and the like, while ensuring a reasonable benefit/risk ratio. Pharmaceutically acceptable salts include nontoxic acid addition salts formed with inorganic acids (such as hydrochloric acid, hydrobromic acid, phosphoric acid, sulfuric acid, and perchloric acid) or organic acids (such as acetic acid, maleic acid, tartaric acid, citric acid, succinic acid, or malonic acid), as well as alkali or alkaline earth metal salts (such as sodium, lithium, potassium, calcium, magnesium, and the like).

Prevent or prevention: As used herein, these terms refer to reducing the risk of developing a disease, disorder, and/or condition, and/or delaying the onset of one or more symptoms of the disease, disorder, or condition. Prevention may be considered complete when the onset of a disease, disorder, or condition has been delayed for a predefined period.

Protein: As used herein, the term "protein" refers to a polypeptide (i.e., a string of at least two amino acids linked together by peptide bonds). Proteins may include moieties other than amino acids (such as glycoproteins or proteoglycans) and/or may be otherwise processed or modified.

Polypeptide: The term "polypeptide," as used herein, generally refers to a polymer of at least three amino acids. The term encompasses not only polypeptides having a complete sequence recited herein, but also functional fragments of such polypeptides.

Prevention: The term "prevention" as used herein refers to a delay of onset, and/or reduction in frequency and/or severity of one or more symptoms of a particular disease, disorder, or condition. Prevention may be considered complete when the onset of a disease, disorder, or condition has been delayed for a predefined period.

Pure: As used herein, an agent or entity is "pure" if it is substantially free of other components. A preparation that contains more than about 90% of a particular agent or entity is typically considered to be a pure preparation.

Reference: As used herein, this term describes a standard or control relative to which a comparison is performed. In some embodiments, a reference or control is tested and/or determined substantially simultaneously with the testing or determination of interest.

Sample: As used herein, the term "sample" typically refers to an aliquot of material obtained or derived from a source of interest, which can be biological or environmental. A biological tissue or fluid sample may be obtained, for example, by aspirate, biopsy, swab, surgery, or lavage.

Stable nanoparticle composition: As used herein, the term "stable" means that the compositions maintain one or more aspects of their physical structure (such as size range and/or distribution of particles) over a period of time. A stable composition may retain its biologically relevant activity for a defined period.

Subject: As used herein, the term "subject" refers to an organism, typically a mammal (for example, a human). A subject may be suffering from, susceptible to, or at risk for a disease, disorder, or condition.

Substantially: As used herein, the term "substantially" refers to total or near-total extent or degree of a characteristic or property of interest, capturing the potential lack of completeness inherent in many biological and chemical phenomena.

Susceptible to: An individual who is "susceptible to" a disease, disorder, or condition is at risk of developing it, which may be a population-based risk.

Systemic: The phrases "systemic administration," "administered systemically," "peripheral administration," and "administered peripherally," as used herein, refer to the administration of a compound or composition such that it enters the recipient's system.

Therapeutic agent: As used herein, the phrase "therapeutic agent" refers to an agent that, when administered to a subject, provides a therapeutic effect.

Therapeutically effective amount: As used herein, the term "therapeutically effective amount" refers to an amount of a substance sufficient to achieve the desired biological response in a therapeutic regimen.

"Tissue" and/or "organ": As used herein, the term "tissue" and/or "organ" refers to viable cellular materials in aggregate form, as well as dispersed cells.

Treatment: As used herein, the term "treatment" refers to the administration of a therapy that alleviates, ameliorates, relieves, inhibits, delays the onset of, reduces the severity of, or reduces the incidence of one or more symptoms of a particular disease, disorder, or condition. Treatment may be prophylactic or therapeutic.

### EXPERIMENTAL SECTION

The ¹H-NMR data of selected compounds are listed in the form of ¹H-NMR peaklists. Therein, for each signal peak the δ value in ppm is given, followed by the signal intensity, reported in round brackets. The δ value-signal intensity pairs from different peaks are separated by commas. Therefore, a peaklist is described by the general form: δ₁ (intensity₁), δ₂ (intensity₂), ... , δᵢ (intensityᵢ), ... , δₙ (intensityₙ).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A ¹H-NMR peaklist is similar to a classical ¹H-NMR readout, and thus usually contains all the peaks listed in a classical NMR interpretation. Moreover, similar to classical ¹H-NMR printouts, peaklists can show solvent signals, signals derived from stereoisomers of the particular target compound, peaks of impurities, ¹³C satellite peaks, and/or spinning sidebands. The peaks of stereoisomers, and/or peaks of impurities are typically displayed with a lower intensity compared to the peaks of the target compound (e.g., with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical ¹H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

Chemical names were generated using the ACD/Name software from ACD/Labs. In some cases generally accepted names of commercially available reagents were used in place of ACD/Name generated names.

The following table lists the abbreviations used in this paragraph and in the Examples section as far as they are not explained within the text body. Other abbreviations have their meanings customary *per se* to the skilled person.

The various aspects of the invention described in this application are illustrated by the following examples which are not meant to limit the invention in any way.

The example testing experiments described herein serve to illustrate the present invention and the invention is not limited to the examples given.

### THE FOLLOWING SECTIONS COVER METHODS of SYNTHESIS, INTERMEDIATES OF THE IONIZABLE LIPIDS OF FORMULA I:

The compounds according to the invention of general formula (I) can be prepared according to the following synthesis scheme. The procedure described below illustrates one synthetic route to the compounds of general formula (I) of the invention and is not intended to be limiting. It is clear to the person skilled in the art that the order of transformations can be modified in various ways. The order of transformations exemplified in this scheme is therefore not intended to be limiting. In addition, interconversion of any of the substituents, R, and R1 can be achieved before and/or after the exemplified transformations. These modifications can be such as the introduction of protecting groups, cleavage of protecting groups, reduction or oxidation of functional groups, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art (see for example T.W. Greene and P.G.M. Wuts in Protective Groups in Organic Synthesis, 3rd edition, Wiley 1999). Specific examples are described in the subsequent paragraphs.

An exemplary route for the preparation of compounds of general formula (I), including intermediates- in which R¹, R², Q, X and Z have the meaning as given for general formula (I), *supra - is shown below:*

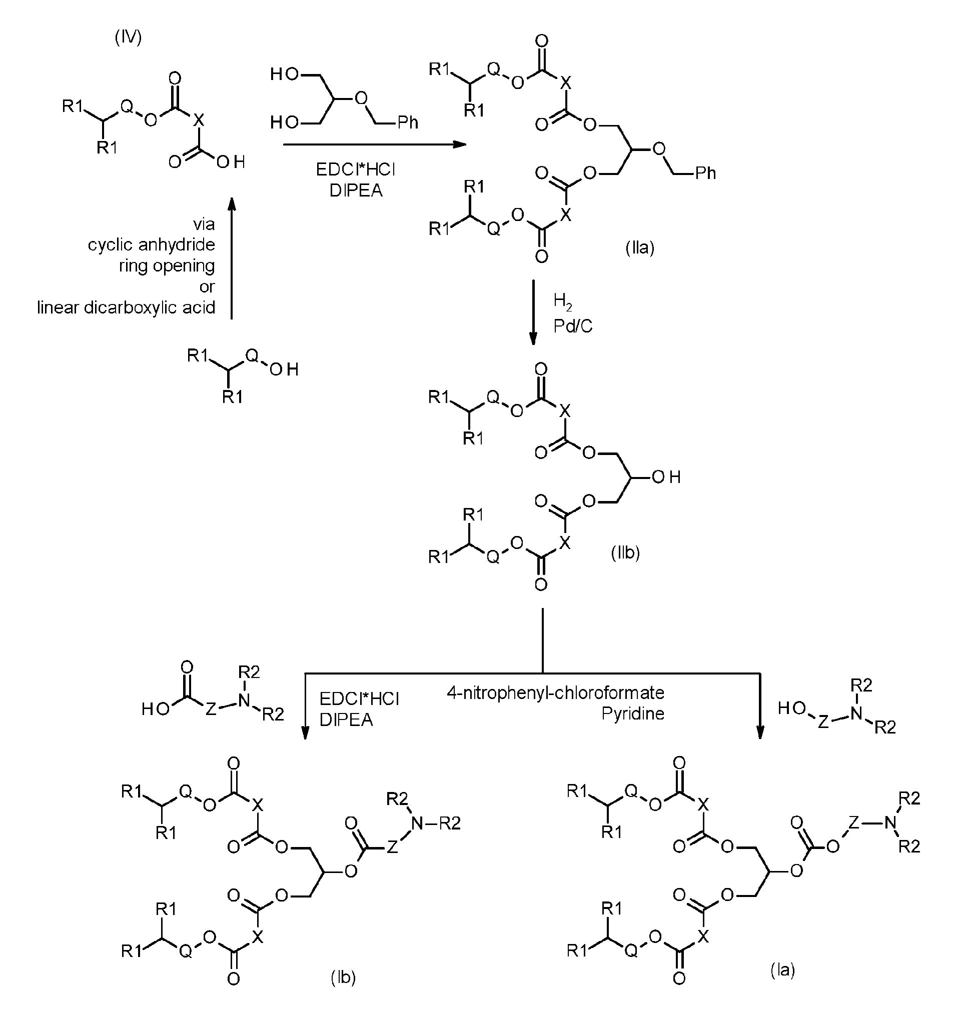

Compounds other than those secribed under general formulas (I), (Ia), (Ib), (IIa), and (IIb) are either commercially available or can be prepared according to procedures available from the public domain, as understandable to the person skilled in the art. Specific examples are described in the Experimental Section.

In accordance with a second aspect, the present invention covers methods of preparing compounds of general formula (I) as defined *supra,* said methods comprising the step of allowing an intermediate compound of general formula (Ila) and (Ilb) as outlined in Scheme 1.

In accordance with a third aspect, the present invention covers methods of preparing compounds of general formula (I) as defined *supra,* said methods comprising the step of allowing an intermediate compound of general formula (IIa) or (IIb) :
in which R¹ and X are as defined for the compound of general formula (I) as defined *supra,*
to react with a compound of general formula (IIIa) or (IIIb) as defined in Scheme 1, in which R¹and X are as defined for the compound of general formula (I) as defined *supra,* 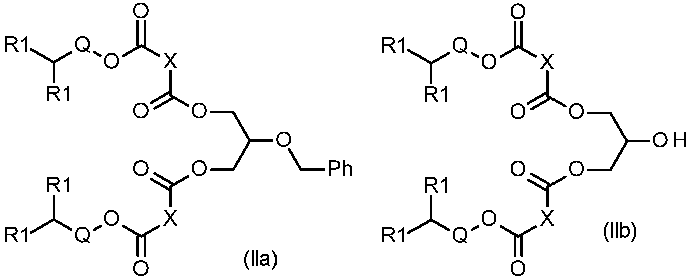
thereby giving a compound of general formula (I), (Ia) or (Ib) : in which R¹, R², Q, X and Z are as defined *supra,*
then optionally converting said compound into solvates, salts and/or solvates of such salts using the corresponding (i) solvents and/or (ii) bases or acids.

The present invention covers methods of preparing compounds of the present invention of general formula (I), said methods comprising the steps as described in the Experimental Section herein.

In accordance with a fourth aspect, the present invention covers intermediate compounds which are useful for the preparation of the compounds of general formula (I), *supra.*

Particularly, the inventions covers the intermediate compounds of general formula (Ila) and (Ilb) :

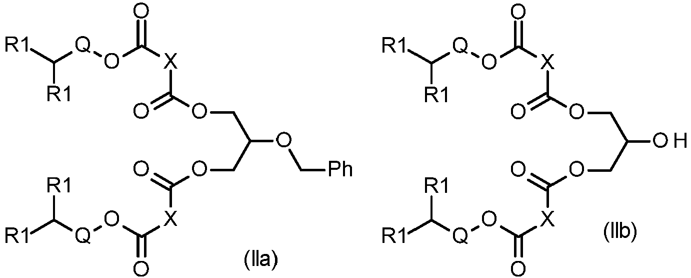

in which R¹ and X are as defined for the compound of general formula (I) *supra.*

In accordance with a fifth aspect, the present invention covers the use of said intermediate compounds for the preparation of a compound of general formula (I) as defined *supra.*

Particularly, the inventions covers the use of intermediate compounds of general formula (lla) and (IIb) in which R¹ and X are as defined for the compound of general formula (I) *supra,* for the preparation of a compound of general formula (I) as defined *supra.*

The present invention covers the intermediate compounds which are disclosed in the Example Section of this text, *infra.*

The present invention covers any sub-combination within any embodiment or aspect of the present invention of intermediate compounds of general formula (I), (IIa), (IIb), (IIIa), (IIIb) *supra.*

The compounds of general formula (I) of the present invention can be converted to any salt, preferably pharmaceutically or functionally acceptable salts, as described herein, by any method which is known to the person skilled in the art. Similarly, any salt of a compound of general formula (I) of the present invention can be converted into the free compound, by any method which is known to the person skilled in the art.

Compounds of general formula (I) of the present invention demonstrate a valuable spectrum of action, in particular in the context of lipid nanoparticle formation and function as well as drug delivery if supported by data, both of which could not have been predicted. Compounds of the present invention have surprisingly been found to effectively integrate into lipid nanparticles that exhibit a high degree of cargoi or drug delivery to cells

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil^{®} or KP-NH^{®} in combination with a Biotage autopurifier system (SP4^{®} or Isolera Four^{®}) and eluents such as gradients of hexane/ethyl acetate or dichloromethane /methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

### UPLC Standard Procedures

Analytical UPLC was performed as described below. The masses (m/z) are reported from the positive mode electrospray ionisation unless the negative mode is indicated (ESI-). In most of the cases method 1 is used. If not, it is indicated.

### Method 1:

System MS: Waters TOF instrument; System UPLC: Waters Acquity I-CLASS; Column: Waters, XSELECT CSH C18, 4.6 x 50 mm 3.5 µm; Eluent A: 5 mM ammonium acetate pH 4.2; Eluent B: 100 mM ammonium acetate pH 4.2/lsopropanol/Acetonitrile (5/62/33 v/v); Gradient: 0.0 min 85% B → 2.0 min 85% B → 19.0 min 100% B → 24.0 min 95% B; Oven: 50 °C; Flow: 0.75 mL/min; UV-Detection: 240 nm.

### Method 2:

System MS: Thermo Scientific FT-MS; System UHPLC+: Thermo Scientific Vanquish; Column: Waters, HSST3, 2.1 x 75 mm, C18 1.8 µm; Eluent A: 1 L Water + 0.01% Formic acid; Eluent B: 1 L Acetonitrile + 0.01% Formic acid; Gradient: 0.0 min 60% B → 0.75 min 95% B → 3.5 min 95% B; Oven: 50 °C; Flow: 1.00 mL/min; UV-Detection: 210 nm/ Optimum Integration Path 210-300 nm.

### Method 3:

System MS: Thermo Scientific FT-MS; System UHPLC+: Thermo Scientific UltiMate 3000; Column: Waters, HSST3, 2.1 x 75 mm, C18 1.8 µm; Eluent A: 1 L Water + 0.01% Formic acid; Eluent B: 1 L Acetonitrile + 0.01% Formic acid; Gradient: 0.0 min 10% B → 2.5 min 95% B → 3.5 min 95% B; Oven: 50 °C; Flow: 0.90 mL/min; UV-Detection: 210 nm/ Optimum Integration Path 210-300 nm.

### Example 1

### 1,1'-diheptadecan-9-yl 5,5'-(2-{[4-(dimethylamino)butanoyl]oxy}propane-1,3-diyl) dipentanedioate

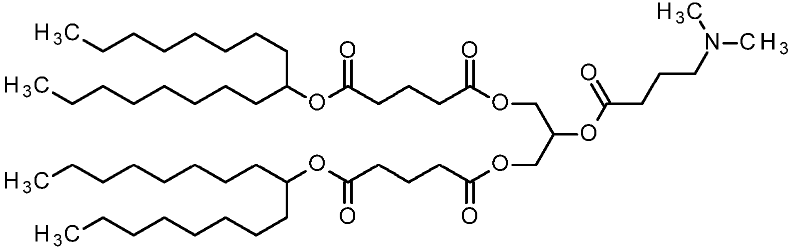

Under Argon, 4-(dimethylamino)butanoic acid hydrochloride (1.26 g, 7.53 mmol) was dissolved in dichloromethane (50 mL). To this, 4-(dimethylamino)pyridine (766 mg, 6.27 mmol) and N,N-diisopropylethylamine (2.73 mL, 15.7 mmol) were added, followed by 1-Ethyl-3-(3-dimethylaminopropyl)carbodiimide hydrochloride (1.44 g, 7.53 mol), and the mixture was stirred for 30 min at RT. After this time, 1,1'-diheptadecan-9-yl 5,5'-(2-hydroxypropane-1,3-diyl) dipentanedioate (5.00 g, 6.27 mmol) was added and the mixture was further stirred overnight at RT. The reaction mixture was concentrated and purified by flash column chromatography (Biotage Selekt; column: SFär-cartridge; eluant: gradient petroleum ether/Ethyl acetate 50% to 100%). Product was obtained as a light yellow oil: 1.04 g (98 % purity, 18 % yield).
LC-MS (EC14189-9-P1B): Rt = 2.99 min; MS (ESlpos): m/z = 911 [M+H]⁺

¹H-NMR (400 MHz, CHLOROFORM-d) δ [ppm]: 5.32 - 5.21 (m, 1H), 4.87 (quin, J = 6.3 Hz, 2H), 4.32 (dd, J = 4.3, 11.9 Hz, 2H), 4.16 (dd, J = 5.9, 11.9 Hz, 2H), 2.48 - 2.26 (m, 12H), 2.22 (s, 6H), 1.95 (quin, J = 7.4 Hz, 4H), 1.83 - 1.75 (m, 2H), 1.51 (br d, *J* = 5.8 Hz, 8H), 1.36 - 1.18 (m, 48H), 0.99 - 0.79 (m, 12H).

### Example 1

### 1,1'-diheptadecan-9-yl 5,5'-(2-{[4-(pyrrolidin-1-yl)butanoyl]oxy}propane-1,3-diyl) dipentanedioate

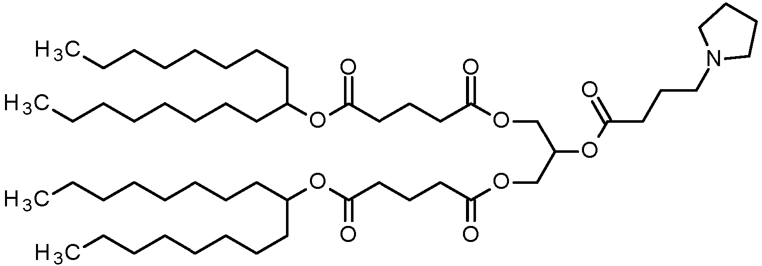

Under Argon, 4-(pyrrolidin-1-yl)butanoic acid hydrochloride (76.7 mg, 95 % purity, 376 µmol) was dissolved in dichloromethane (3.1 mL). To this, 4-(dimethylamino)pyridine (6.13 mg, 50.2 µmol) and N,N-diisopropylethylamine (140 µL, 800 µmol) were added, followed by 1-Ethyl-3-(3-dimethylaminopropyl)carbodiimide hydrochloride (101 mg, 527 µmol), and the mixture was stirred for 30 min at RT. After this time, 1,1'-diheptadecan-9-yl 5,5'-(2-hydroxypropane-1,3-diyl) dipentanedioate (200 mg, 251 µmol) was added and the mixture was further stirred overnight at RT. The reaction mixture was concentrated and purified by flash column chromatography (Biotage Selekt; column: 11 g SFär Amino-cartridge; eluant: gradient Cyclohexane/Ethyl acetate). Fractions were analyzed by TLC (using amino-modified silica plates, with eluant Cyclohexane/Ethyl acetate 8:2 + KMnO₄-stain, Rf-value: 0.17). Product was obtained as a light yellow oil: 189 mg (98 % purity, 79 % yield).
LC-MS (Method 1): Rt = 9.43 min; MS (ESlpos): m/z = 937 [M+H]⁺

¹H-NMR (400 MHz, CHLOROFORM-d) δ [ppm]: 0.860 (2.87), 0.878 (6.95), 0.894 (2.52), 1.257 (16.00), 1.295 (1.54), 1.313 (0.64), 1.497 (1.32), 1.511 (1.25), 1.749 (0.46), 1.757 (0.71), 1.766 (1.39), 1.775 (0.73), 1.783 (0.52), 1.813 (0.43), 1.831 (0.63), 1.850 (0.47), 1.920 (0.88), 1.939 (1.38), 1.957 (1.00), 2.329 (1.18), 2.347 (2.12), 2.367 (1.38), 2.372 (1.47), 2.390 (2.29), 2.409 (1.10), 2.435 (0.63), 2.455 (0.83), 2.473 (1.02), 2.485 (1.20), 4.126 (0.47), 4.140 (0.52), 4.155 (0.68), 4.170 (0.66), 4.290 (0.66), 4.301 (0.69), 4.320 (0.50), 4.331 (0.48), 4.849 (0.46), 4.864 (0.66), 4.880 (0.45).

### Example 2

### 1,1'-(2-{[5-(dimethylamino)pentanoyl]oxy}propane-1,3-diyl) 5,5'-diheptadecan-9-yl dipentanedioate

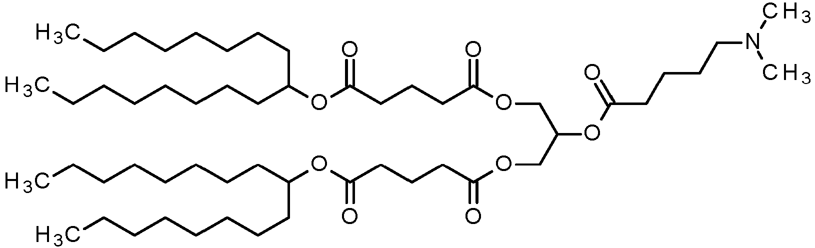

Under Argon, 5-(dimethylamino)pentanoic acid (32.8 mg, 376 µmol) was dissolved in dichloromethane (3.1 mL). To this, 4-(dimethylamino)pyridine (6.13 mg, 50.2 µmol) and N,N-diisopropylethylamine (140 µL, 800 µmol) were added, followed by 1-Ethyl-3-(3-dimethylaminopropyl)carbodiimide hydrochloride (101 mg, 527 µmol), and the mixture was stirred for 30 min at RT. After this time, 1,1'-diheptadecan-9-yl 5,5'-(2-hydroxypropane-1,3-diyl) dipentanedioate (200 mg, 251 µmol) was added and the mixture was further stirred overnight at RT. The reaction mixture was concentrated and purified by flash column chromatography (Biotage Selekt; column: 11 g KP-NH-cartridge; eluant: gradient Cyclohexane/Ethyl acetate), followed by a second flash cholumn chromatography (Biotage Selekt; column: 10 g SFär-cartridge; eluant: isocratic Ethyl acetate/MeOH 10:1 v/v). Fractions were analyzed by TLC (using amino-modified silica plates, with eluant Cyclohexane/Ethyl acetate 8:2 + KMnO₄-stain, Rf-value: 0.14). Product was obtained as a light yellow oil: 142 mg (98 % purity, 64 % yield).
LC-MS (Method 1): Rt = 9.71 min; MS (ESlpos): m/z = 925 [M+H]⁺

¹H-NMR (400 MHz, CHLOROFORM-d) δ [ppm]: 0.860 (2.71), 0.878 (6.35), 0.894 (2.44), 1.257 (16.00), 1.296 (1.52), 1.313 (0.63), 1.499 (1.59), 1.511 (1.45), 1.650 (0.48), 1.920 (0.87), 1.939 (1.39), 1.957 (1.01), 2.218 (6.99), 2.259 (0.50), 2.277 (0.64), 2.295 (0.46), 2.330 (1.22), 2.336 (0.81), 2.349 (2.24), 2.355 (1.33), 2.367 (1.16), 2.374 (1.77), 2.392 (2.00), 2.411 (0.95), 4.123 (0.47), 4.138 (0.50), 4.153 (0.66), 4.167 (0.64), 4.292 (0.65), 4.303 (0.68), 4.322 (0.49), 4.333 (0.48), 4.849 (0.45), 4.865 (0.65), 4.880 (0.44).

### Example 3

### 1,1'-(2-{[4-(dimethylamino)butanoyl]oxy}propane-1,3-diyl) 5,5'-bis(2-octyldecyl) dipentanedioate

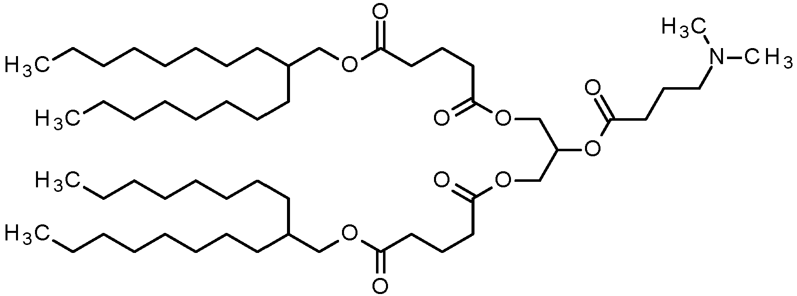

Under Argon, 4-(dimethylamino)butanoic acid hydrochloride (64.1 mg, 95 % purity, 364 µmol) was dissolved in dichloromethane (3.0 mL). To this, 4-(dimethylamino)pyridine (5.92 mg, 48.5 µmol) and N,N-diisopropylethylamine (140 µL, 780 µmol) were added, followed by 1-Ethyl-3-(3-dimethylaminopropyl)carbodiimide hydrochloride (97.6 mg, 509 µmol), and the mixture was stirred for 15 min at RT. After this time, 1,1'-(2-hydroxypropane-1,3-diyl) 5,5'-bis(2-octyldecyl) dipentanedioate (200 mg, 242 µmol) was added and the mixture was further stirred for 2.5 days at RT. The reaction mixture was concentrated and purified by flash column chromatography (Biotage Selekt; column: 11 g SFär-Amino-cartridge; eluant: gradient Cyclohexane/Ethyl acetate). Fractions were analyzed by TLC (using amino-modified silica plates, with eluant Cyclohexane/Ethyl acetate 8:2 + KMnO₄-stain, Rf-value: 0.17). Product was obtained as a colorless oil: 165 mg (98 % purity, 71 % yield).
LC-MS (Method 1): Rt = 11.60 min; MS (ESlpos): m/z = 939 [M+H]⁺

¹H-NMR (400 MHz, CHLOROFORM-d) δ [ppm]: 0.864 (1.92), 0.882 (4.47), 0.898 (1.73), 1.264 (16.00), 1.300 (1.04), 1.318 (0.42), 1.783 (0.49), 1.922 (0.64), 1.940 (1.02), 1.959 (0.73), 2.208 (5.73), 2.261 (0.43), 2.280 (0.65), 2.352 (1.04), 2.370 (2.04), 2.377 (1.11), 2.389 (1.12), 2.396 (1.56), 2.415 (0.69), 3.966 (1.45), 3.980 (1.44), 4.155 (0.47), 4.170 (0.47), 4.288 (0.46), 4.299 (0.49).

### Example 4

### 1,1'-bis(2-octyldecyl) 5,5'-(2-{[4-(pyrrolidin-1-yl)butanoyl]oxy}propane-1,3-diyl) dipentanedioate

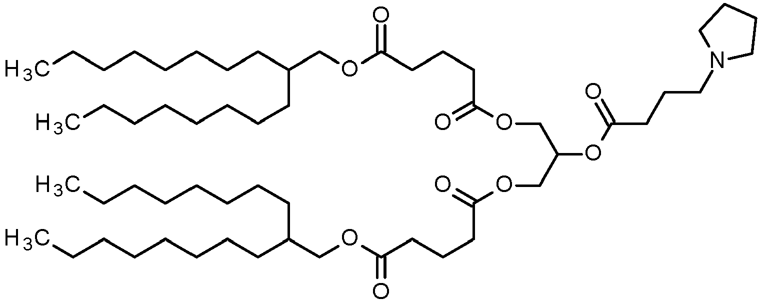

Under Argon, 4-(pyrrolidin-1-yl)butanoic acid hydrochloride (74.1 mg, 95 % purity, 364 µmol) was dissolved in dichloromethane (3.0 mL). To this, 4-(dimethylamino)pyridine (5.92 mg, 48.5 µmol) and N,N-diisopropylethylamine (140 µL, 780 µmol) were added, followed by 1-Ethyl-3-(3-dimethylaminopropyl)carbodiimide hydrochloride (97.6 mg, 509 µmol), and the mixture was stirred for 15 min at RT. After this time, 1,1'-(2-hydroxypropane-1,3-diyl) 5,5'-bis(2-octyldecyl) dipentanedioate (200 mg, 242 µmol) was added and the mixture was further stirred for 2.5 days at RT. The reaction mixture was concentrated and purified by flash column chromatography (Biotage Selekt; column: 11 g SFär-Amino-cartridge; eluant: gradient Cyclohexane/Ethyl acetate). Fractions were analyzed by TLC (using amino-modified silica plates, with eluant Cyclohexane/Ethyl acetate 8:2 + KMnO₄-stain, Rf-value: 0.17). Product was obtained as a colorless oil: 187 mg (98 % purity, 78 % yield).

¹H-NMR (400 MHz, CHLOROFORM-d) δ [ppm]: 0.007 (0.64), 0.870 (2.12), 0.888 (5.33), 0.905 (1.87), 1.270 (16.00), 1.308 (1.07), 1.325 (0.45), 1.764 (0.56), 1.773 (1.12), 1.781 (0.59), 1.790 (0.42), 1.837 (0.50), 1.928 (0.72), 1.946 (1.15), 1.965 (0.82), 2.359 (0.93), 2.377 (2.02), 2.383 (1.23), 2.395 (1.55), 2.402 (1.84), 2.412 (0.58), 2.421 (0.81), 2.442 (0.51), 2.461 (0.67), 2.479 (0.81), 2.491 (0.96), 3.972 (1.63), 3.987 (1.63), 4.145 (0.41), 4.160 (0.55), 4.175 (0.54), 4.294 (0.53), 4.305 (0.58), 4.323 (0.40), 4.334 (0.40).

### Intermediate 1

### 1,1'-diheptadecan-9-yl 5,5'-(2-hydroxypropane-1,3-diyl) dipentanedioate

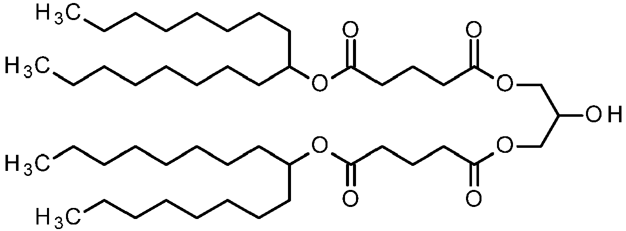

Under Argon, 1,1'-[2-(benzyloxy)propane-1,3-diyl] 5,5'-diheptadecan-9-yl dipentanedioate (1.03 g, 1.16 mmol) was dissolved in THF (13 mL) and then treated with Pd/C 10% wt (98.2 mg). The reaction mixture was switched to a H₂-atmosphere at normal pressure and further stirred at RT overnight. After conversion control by TLC (silica, Cyclohexane-Ethyl acetate 8:2 + KMnO₄-stain: benzyl ether fully consumed, product with Rf-value: 0.25), the reaction mixture was filtered over Celite, washed with THF and concentrated, yielding the product as a light yellow oil: 903 mg (98 % purity, 96 % yield).
LC-MS (Method 1): Rt = 8.59 min; MS (ESlpos): m/z = 815 [M+H]⁺

¹H-NMR (400 MHz, CHLOROFORM-d) δ [ppm]: 0.860 (2.80), 0.878 (6.68), 0.895 (2.46), 1.257 (16.00), 1.296 (1.51), 1.314 (0.63), 1.499 (1.33), 1.513 (1.26), 1.948 (0.98), 1.966 (1.52), 1.985 (1.06), 2.345 (1.18), 2.363 (2.20), 2.381 (1.00), 2.408 (1.17), 2.426 (2.15), 2.445 (0.97), 4.117 (0.60), 4.145 (0.99), 4.160 (0.64), 4.180 (0.83), 4.190 (0.61), 4.853 (0.45), 4.868 (0.65), 4.884 (0.44).

### Intermediate 2

### 1,1'-[2-(benzyloxy)propane-1,3-diyl] 5,5'-diheptadecan-9-yl dipentanedioate

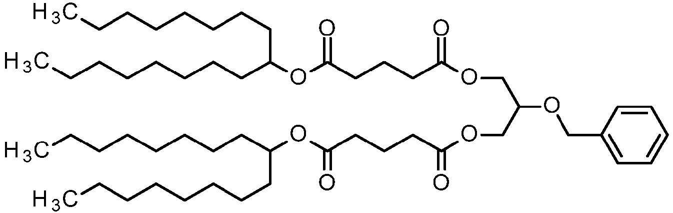

Under Argon, 2-(benzyloxy)propane-1,3-diol (250 mg, 1.37 mmol) and 5-[(heptadecan-9-yl)oxy]-5-oxopentanoic acid (1.14 g, 3.09 mmol) were dissolved in dichloromethane (16 mL). The mixture was then treated with 4-(dimethylamino)pyridine (67.0 mg, 549 µmol), N,N-diisopropylethylamine (660 µL, 3.8 mmol) and 1-Ethyl-3-(3-dimethylaminopropyl)carbodiimide hydrochloride (658 mg, 3.43 mmol), and further stirred at RT. Conversion control by TLC (silica, petroleum ether- Methyl *tert*-butyl ether 4:1, KMnO₄-stain) showed full conversion of diol. The mixture was diluted with dichloromethane (30 mL), and washed with satd. aq. NH₄Cl (1x) and with brine (1x), then dried over MgSO₄, filtered and concentrated. The obtained residue was purified by flash column chromatography (Biotage Selekt; column: 25 g SFär-cartridge; eluant: gradient Cyclohexane-Ethyl acetate 1% to 30%) and the tubes containing product were combined and concentrated, yielding the desired product as a colorless oil: 1.03 g (99 % purity, 84 % yield).
LC-MS (Method 1): Rₜ = 10.72 min; MS (ESlpos): m/z = 905 [M+NH₄]⁺

¹H-NMR (400 MHz, CHLOROFORM-d) δ [ppm]: 0.858 (2.88), 0.876 (6.92), 0.892 (2.57), 1.254 (16.00), 1.294 (1.62), 1.311 (0.67), 1.496 (1.37), 1.510 (1.28), 1.921 (0.95), 1.939 (1.50), 1.957 (1.08), 2.327 (1.26), 2.346 (2.27), 2.365 (2.20), 2.384 (2.19), 2.402 (1.04), 4.133 (0.49), 4.147 (0.47), 4.162 (0.84), 4.176 (0.76), 4.235 (0.83), 4.246 (0.79), 4.264 (0.50), 4.276 (0.46), 4.644 (2.64), 4.852 (0.48), 4.867 (0.68), 4.883 (0.46), 7.263 (0.89), 7.333 (3.64), 7.342 (0.92), 7.347 (0.84).

### Intermediate 3

### 5-[(heptadecan-9-yl)oxy]-5-oxopentanoic acid

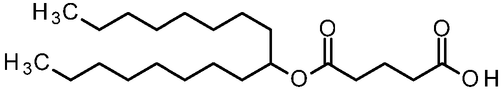

Under Argon, heptadecan-9-ol (1.00 g, 3.90 mmol) was dissolved in dichloromethane (30 mL). To this solution, triethylamine (1.1 mL, 7.8 mmol), 4-(dimethylamino)pyridine (953 mg, 7.80 mmol) and glutaric anhydride (2.67 g, 23.4 mmol) were added and the mixture was further stirred at RT overnight. After this time, the reaction was quenched by adding aq. HCl 1 N (25 mL) and then extracted with dichloromethane (3x). The combined organic layers were washed with brine (1x), dried over Na₂SO₄, filtered and concentrated. The obtained residue was further purified by flash column chromatography (Biotage Selekt; column: 50 g SFär-cartridge; eluant: gradient Cyclohexane-Ethyl acetate 2% to 30%), controlling collected fractions by TLC (using phosphomolybdic acid-stain). The desired product eluted at ~10-15% Ethyl acetate and, after concentrating, it was obtained as a very light yellow liquid: 1.33 g (98 % purity, 90 % yield).
LC-MS (Method 2): Rt = 1.36 min; MS (ESlneg): m/z = 369 [M-H]⁻

¹H-NMR (400 MHz, CHLOROFORM-d) δ [ppm]: 0.860 (3.21), 0.877 (7.90), 0.894 (2.69), 1.258 (16.00), 1.296 (1.72), 1.314 (0.72), 1.516 (1.27), 1.941 (1.06), 1.960 (1.70), 1.978 (1.19), 2.362 (1.37), 2.380 (2.50), 2.399 (1.14), 2.413 (1.36), 2.431 (2.51), 2.450 (1.14), 4.863 (0.49), 4.878 (0.71), 4.894 (0.49), 7.268 (0.73).

### Intermediate 4

### 1,1'-(2-hydroxypropane-1,3-diyl) 5,5'-bis(2-octyldecyl) dipentanedioate

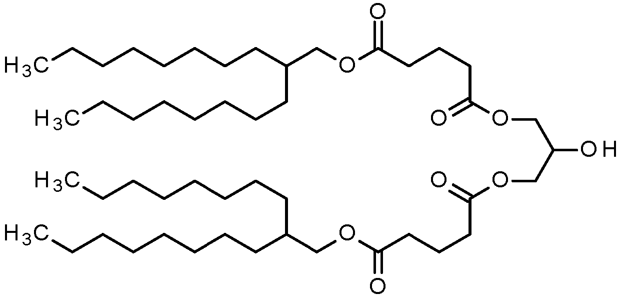

Under Argon, 1,1'-[2-(benzyloxy)propane-1,3-diyl] 5,5'-bis(2-octyldecyl) dipentanedioate (800 mg, 874 µmol) was dissolved in THF (9.6 mL) and then treated with Pd/C 10% wt (73.9 mg). The reaction mixture was switched to a H₂-atmosphere at normal pressure and further stirred at RT overnight. After conversion control by TLC (silica, Cyclohexane-Ethyl acetate 8:2 + KMnO₄-stain: benzyl ether fully consumed, product with Rf-value: 0.21), the reaction mixture was filtered over Celite, washed with THF and concentrated, yielding the product as a colorless oil: 698 mg (98 % purity, 95 % yield).
LC-MS (Method 1): Rt = 10.77 min; MS (ESlpos): m/z = 843 [M+NH₄]⁺

¹H-NMR (400 MHz, CHLOROFORM-d) δ [ppm]: 0.864 (2.14), 0.882 (5.26), 0.898 (1.83), 1.242 (1.11), 1.264 (16.00), 1.301 (1.00), 1.319 (0.40), 1.432 (2.03), 1.949 (0.77), 1.967 (1.25), 1.985 (0.87), 2.045 (1.20), 2.367 (0.95), 2.385 (1.81), 2.403 (0.83), 2.413 (1.00), 2.431 (1.82), 2.449 (0.80), 3.969 (1.66), 3.984 (1.64), 4.116 (0.56), 4.144 (0.82), 4.158 (0.49), 4.179 (0.64), 4.189 (0.48).

### Intermediate 5

### 1,1'-[2-(benzyloxy)propane-1,3-diyl] 5,5'-bis(2-octyldecyl) dipentanedioate

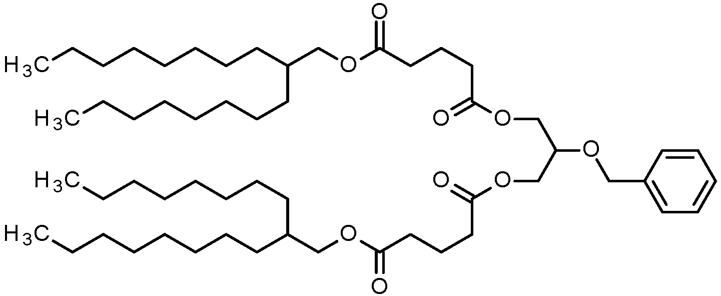

Under Argon, 2-(benzyloxy)propane-1,3-diol (165 mg, 904 µmol) and 5-[(2-octyldecyl)oxy]-5-oxopentanoic acid (782 mg, 2.03 mmol) were dissolved in dichloromethane (9.0 mL). The mixture was then treated with 4-(dimethylamino)pyridine (44.2 mg, 361 µmol), N,N-diisopropylethylamine (430 µL, 2.5 mmol) and 1-Ethyl-3-(3-dimethylaminopropyl)carbodiimide hydrochloride (433 mg, 2.26 mmol), and further stirred at RT. Conversion control by TLC (silica, Cyclohexane-Ethyl acetate 85:15, PMA-stain) showed full conversion of diol. The mixture was diluted with dichloromethane (30 mL), and washed with satd. aq. NH₄Cl (1x) and with brine (1x), then dried over Na₂SO₄, filtered and concentrated. The obtained residue was purified by flash column chromatography (Biotage Selekt; column: 25 g SFär-cartridge; eluant: gradient Cyclohexane-Ethyl acetate 2% to 10%) and the tubes containing product were combined and concentrated, yielding the desired product (805 mg (98 % purity, 95 % yield)) as a colorless liquid.

¹H-NMR (400 MHz, CHLOROFORM-d) δ [ppm]: 0.009 (0.64), 0.871 (2.17), 0.889 (5.45), 0.905 (1.88), 1.270 (16.00), 1.308 (1.05), 1.325 (0.44), 1.929 (0.75), 1.947 (1.20), 1.966 (0.85), 2.357 (0.96), 2.376 (2.18), 2.396 (2.11), 2.415 (0.81), 3.973 (1.68), 3.987 (1.66), 4.169 (0.66), 4.183 (0.60), 4.242 (0.64), 4.254 (0.62), 4.651 (2.00), 7.342 (2.74), 7.349 (0.66), 7.356 (0.55).

### Intermediate 6

### 5-[(2-octyldecyl)oxy]-5-oxopentanoic acid

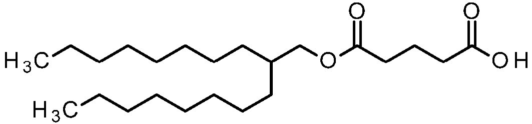

2-octyldecan-1-ol (1.55 g, 5.73 mmol) was dissolved in toluene (9.5 mL). To this, glutaric anhydride (687 mg, 6.02 mmol) and 4-(dimethylamino)pyridine (840 mg, 6.88 mmol; CAS-RN:[1122-58-3]) were added in one portion at RT and the mixture was then stirred overnight (~18 h reaction) under reflux. After this time, the mixture was cooled down to RT and analyzed by TLC (silica; Cyclohexane-Ethyl acetate 15%, + phosphomolybdic acid-stain): 2-octyldecan-1-ol was barely visible (Rf ~ 0.6), along with a strong, tailing spot near the baseline (Rf ~ 0.1-0.3). The reaction mixture was diluted with dichloromethane (50 mL) and washed with aq. HCl 1 N (2 x 30 mL), then with water (2 x 30 mL) and brine (1 x 30 mL). The organic phase was dried over Na₂SO₄, filtered and concentrated to yield a light yellow liquid, which was further purified by flash column chromatography (Biotage Selekt; column: 100 g SFär cartridge; eluant: gradient Cyclohexane-Ethyl acetate 5% to 30%). The desired product eluted at ~15% Ethyl acetate, and the tubes containing it were combined, concentrated in rotavap and dried under high-vacuum overnight, yielding a colorless liquid: 783 mg (99 % purity, 35 % yield).
LC-MS (Method 3): Rt = 3.24 min; MS (ESlneg): m/z = 383 [M-H]⁻

¹H-NMR (500 MHz, CHLOROFORM-d) δ [ppm]: 0.867 (3.20), 0.881 (7.01), 0.895 (2.66), 1.262 (16.00), 1.298 (1.36), 1.312 (0.60), 1.427 (1.01), 1.947 (1.13), 1.962 (1.79), 1.977 (1.24), 2.387 (1.33), 2.401 (2.52), 2.416 (1.22), 2.423 (1.40), 2.437 (2.60), 2.452 (1.20), 2.651 (0.57), 3.975 (2.29), 3.987 (2.26).

### Intermediate 7

### 2-octyldecan-1-ol

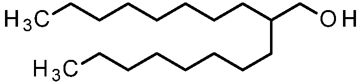

Under Argon, a round-bottom flask was charged with dry THF (22 mL) and then LiAlH4 (7.0 mL, 2.0 M in THF, 14 mmol) was added dropwise. After cooling this to 0 °C (ice-water bath), a solution of 2-octyldecanoic acid (2.00 g, 7.03 mmol) in dry THF (32 mL) was added using an addition funnel. After stirring for 30 min at 0 °C, the cooling bath was removed and the mixture was further stirred under Argon at RT overnight. After this time, the mixture was cooled to 0 °C, quenched by slowly adding 1 mL Ethyl acetate, then slowly adding 1 mL ethanol. After removing the cooling bath, 30 mL of dist. water and 30 mL aq. NaOH 1 N were added and the mixture was stirred for 30 min. The mixture was filtered over Celite and the filtrate was concentrated in rotavap, yielding a yellow biphasic liquid residue. This was diluted with dist. water (100 mL) and Methyl tert-butyl ether (50 mL), and the aqueous layer was extracted with Methyl *tert*-butyl ether (2 x 30 mL). The combined organic layers were washed with aq. NaOH 1 N (1 x 30 mL) and dist. water (1 x 30 mL), then dried over Na₂SO₄, filtered and concentrated. This organic extract showed one main spot on TLC (silica; Cyclohexane-Ethyl acetate 15%; KMnO4-stain and phosphomolybdic acid-stain), at Rf ~ 0.5; and the educt (Rf ~ 0.4) could no longer be detected. This concentrated organic fraction was further purified by flash column chromatography (Biotage Selekt; column: 100 g SFär-cartridge; eluant: Cyclohexane-Ethyl acetate gradient 5% to 30%), using ELSD detection. The main product eluted at ~ 15% Ethyl acetate, and tubes containing this main spot were combined and concentrated, yielding the product as colorless liquid: 1.57 g (98 % purity, 81 % yield).

¹H-NMR (400 MHz, CHLOROFORM-d) δ [ppm]: 0.864 (2.73), 0.882 (6.57), 0.898 (2.50), 1.272 (16.00), 1.427 (0.47), 3.535 (1.08), 7.264 (0.56).

### EXPERIMENTAL SECTION: Stability assay of ionizable lipids

20 mg of the ionizable lipid was dissolved in 1 mL of ethanol and stored at room temperature, in the fridge (4-8 °C) and in the freezer (-20 °C) for 7 and 28 days. The stability of the lipid was detected by UPLC-CAD measurements. Decomposed compounds were evaluated by MS-detection.
**UPLC: Agilent 1290 Infinity II**
**Column: Waters X Select CSH 4.6 x 150 mm, 3.5 µm**
Column-temperature: 40 °C
Flow: 0.9 mL/min
Injection volume: 5 - 10 µL
Runtime: 30 min
Sampler temperature: 12 °C
**CAD-Detector:**
Nebulizer- Temperature: 50 °C
Data Collection Rate: 10 Hz
Power Function: 1.1
Filter Constant: 3.6

### MS-Detector:

HPLC with thermostatic column oven, UV detector, RDA detector (Bioaccord) and data evaluation system: Waters Premier (Quaternary Solvent Manager)
Nebulizer Temperature: 50 °C
Data Collection Rate: 10 Hz
Filter Constant: 3.6
RDa Detector: Esi+

### Mobile Phase:

Mobile Phase A: 5 mM Ammonium acetate pH 4.2
Mobile Phase B: 100 mM Ammonium acetate pH 4.2 / Isopropanol / Acetonitrile (5 / 62 / 33)

### Prepared from the following buffers:

Mobile Phase A: 100 mM Ammonium acetate pH 4.2 LC-MS quality
Mobile Phase B: 3.85 g Ammonium acetate in 500 mL MilliQ water + 7.5 mL glacial acetic acid

### Gradient:

| **Table 2** | | |
|---|---|---|
| min | A [%] | B [%] |
| 0 | 20 | 80 |
| 2 | 20 | 80 |
| 2.1 | 11 | 89 |
| 15 | 8 | 92 |
| 19 | 0 | 100 |
| 24 | 0 | 100 |
| 26 | 20 | 80 |
| 30 | 20 | 80 |

| **Table 3** | | | | | |
|---|---|---|---|---|---|
| **Stability assay (20 mg/mL in Ethanol) % of remaining compound** | | | | | |
| **Example** | **start** | **RT 7d** | **fridge 7d** | **fridge 28 d** | **freezer 28 d** |
| 1 | 92 | 92 | 93 | 93 | 94 |
| 2 | 88 | 22 | 64 | 14 | 77 |
| **3** | 95 | 95 | 95 | 95 | 96 |
| **4** | 93 | 45 | 78 | 34 | 83 |
| **5** | 94 | 22 | 63 | 12 | 72 |

| | | | | | |
|---|---|---|---|---|---|
| * 0.1 mg/mL in Ethanol | | | | | |

### EXPERIMENTAL SECTION - BIOLOGICAL ASSAYS

### Methods for Manufacturing lipid nanoparticles and EPO protein Analysis

### Manufacturing Protocol

For the preparation of the Lipid nanoparticles (LNPs), an ethanolic solution containing the four lipid components of the LNP at a molar ratio of lonizable lipid:DSPC:cholesterol:DMG-PEG2000 of 46:10:42:2 was prepared at a total lipid concentration of 20 mM. The ionizable lipids used were compounds 1, 2, 3, 4, 5 according to the invention. Human erythropoietin (EPO) mRNA was purchased from Trilink, and dissolved in RNAse-free buffer consisting of 10 mM citrate buffer (pH 4) before use. The Lipid nanoparticles (LNPs) were prepared via microfluidics utilizing the NanoAssemblr Ignite System (Precision NanoSystems Inc.) at a flow rate of 12 mL/min and an organic-to-aqueous flow rate ratio of 1:3, yielding LNPs with a fixed amine-to-phosphate (N/P) ratio. The resulting LNPs were dialyzed overnight against a Tris/Sucrose buffer (50 mM Tris, 150 mM Sucrose, pH 7.5) at a 500-fold sample volume using Slide-A-Lizer G3 dialysis cassettes (20000 MWCO, Thermo Scientific) at 4 °C. If required, the LNPs were concentrated via centrifugation using Amicon centrifugal units (2 x 20 min, 5000 g, 4 °C; 50000 MWCO, Millipore). The resulting LNPs were then filtered using a 0.2 µm sterile PES filter (13 mm, Thermo Scientific). RNA concentration and RNA encapsulation were measured using the Ribogreen Assay according to the manufacturer's protocol. Particle size and Zetapotential were measured using dynamic light scattering (DLS) via a Zetasizer Nano Series ZS instrument (Malvern Instruments Ltd.). Upon final characterization, the LNPs were stored at -80 °C until further use.

### In vivo Efficacy

Female CD-1 mice, 18-22 g in body weight were purchased from at Charles River Laboratories. Mice were acclimated for at least 3 days before the initiation of a study. Mice were injected intravenously at a dose of 0.75 mg/kg of human erythropoietin (EPO) mRNA formulated in LNPs. At 4, 8, and 24 h post-injection, plasma was collected for further ELISA analysis. Human Epo levels were measured using hEPO immunoassay from R&D Systems (catalog #DEP00) following manufacturer's recommendations.

Examples were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.

Results for compounds compounds 1, 2, 3, 4, 5 as well as one compound (comparative example) from the prior art are listed in Table 4 and also visualized in Figure 1. Despite the high activity in the in vivo models of LNPs comprising the compounds according to the invention, these LNPs also display higher activities than comparative compounds as Example 13 from WO2015/095340.

| **Table 4** | | | | | |
|---|---|---|---|---|---|
| **No:** | **EPO [mIU/mL] Plasma 4h** | **EPO [mIU/mL] Plasma 8h** | **EPO [mIU/mL] Plasma 24h** | **Size (nm)** | **PDI** |
| 1 | 2099767.0 ± 505745.0 | 2136419.0 ± 849537.4 | | 73.09 | 0.109 |
| 2 | 1837287.4 ± 163189.9 | 1380544.3 ± 505711.6 | 231948.2 ± 131011.4 | 62.81 | 0.122 |
| 3 | 1375826.2 ± 96562.7 | 1257976.1 ± 19167.2 | 176735.4 ± 110207.3 | 69.29 | 0.057 |
| 4 | 1186093.4 ± 307840.4 | 1257143.5 ± 228842.7 | 168016.3 ± 119006.3 | 73.93 | 0.075 |
| 5 | 2094443.0 ± 1158088.1 | 2461053.7 ± 431459.2 | 511620.0 ± 17980.1 | 73.26 | 0.063 |
| Example 13 from WO2015/095340 | 443201.3 ± 138351.4 | 443201.3 ± 147587.6 | | 104.23 | 0.054 |

## Claims

1. A compound of general formula (I) 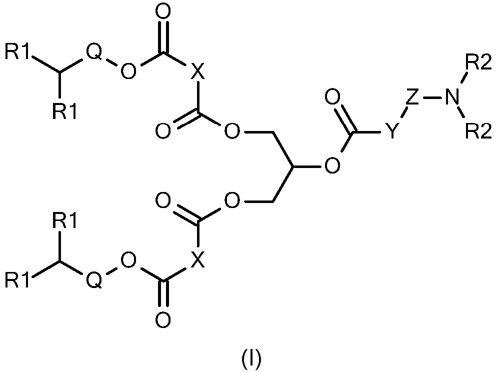 In which
R1 is C₅-C₁₀ alkyl, optionally substituted with 1 to 3 C₁-C₄ alkyl, and or 1-3 methoxy (OMe) or ethoxy (OEt) groups,
Q direct bond, CH₂, C₂-C₅ alkanediyl,
X is C₁-C₄ alkanediyl,
Y is a direct bond, oxygen, nitrogen or sulfur,
Z is C₂-C₈ alkanediyl, optionally substituted with 1 to 3 C₁-C₄ alkyl, and or 1-3 OMe or OEt groups,
R2 is C₁-C₃ alkyl, optionally substituted with 1 to 3 C₁-C₃ alkyl, and or 1-2 OMe or OEt groups, both R2 can also - together with the N they are connected to - form a saturated ring with 4 to 8 members, with 0-3 additional O, N or S in the ring and with 1 to 3 C₁-C₃ alkyl, and or 1-2 OMe or OEt groups, and where the additional substituents can form a second ring, and wherein R2-N-R2 stays symmetrical, and wherein the resulting molecule remains achiral, or a stereoisomer, a tautomer, a hydrate, a solvate, or a salt thereof, or a mixture of same.

2. A compound according to claim 1, in which
R1 is linear or branched C5-C10 alkyl,
Q direct bond, CH2, C2-C5 alkanediyl,
X is C1-C4 alkanediyl,
Y is a direct bond, oxygen, nitrogen or sulfur,
Z is linear or branched C3-C6 alkanediyl
R2 both R2 can also - together with the N they are connected to - form a saturated ring with 4 to 8 members, with 0-3 additional O, N or S in the ring and with 1 to 3 C1-C3 alkyl, and or 1-2 OMe or OEt groups, and where the additional substituents can form a second ring, and wherein R2-N-R2 stays symmetrical, and wherein the resulting molecule remains achiral.

3. A compound according to claim 1 or 2, in which
R1 is linear or branched C₅-C₁₀ alkyl,
Q direct bond, -CH₂-
-X is propane-1,3-diyl
Y is a direct bond, oxygen, nitrogen or sulfur,
Z is linear or branched C₃-C₄ alkanediyl
R2 both R2 can also - together with the N they are connected to - form a saturated ring with 4 to 8 members, with 0-3 additional O, N or S in the ring and with 1 to 3 C₁-C₃ alkyl, and or 1-2 OMe or OEt groups, and where the additional substituents can form a second ring, and wherein R2-N-R2 stays symmetrical, and wherein the resulting molecule remains achiral.

4. A compound according to any of claims 1 to 3, in which
R1 is octyl,
Q direct bond, -CH₂-
-X is propane-1,3-diyl
Y is a direct bond or oxygen,
Z is linear propane-1,3-diyl or butane-1,4-diyl chain,
R2 is C₁-C₃ alkyl, optionally substituted with 1 to 2 C₁-C₃ alkyl, both R2 can also - together with the N they are connected to - form a saturated ring with 5 to 6 members, optionally 1 additional O, N in the ring and with 1 to 2 C₁-C₃ alkyl or O-Methyl groups, and where the additional substituents can form a second ring and where R2-N-R2 stays symmetrical and thus the whole molecule remains achiral.

5. A compound of general formula (IIa) or (IIb) 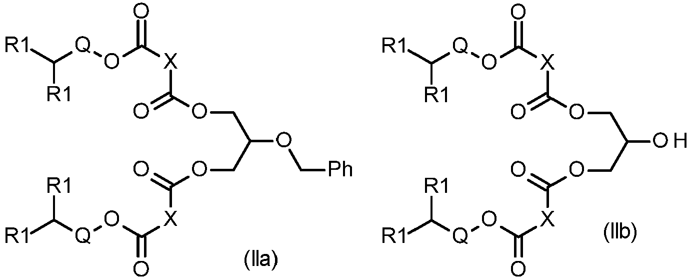 In which
Q direct bond, CH₂, C₂-C₅ alkanediyl,
R1 is C₅-C₁₀ alkyl, optionally substituted with 1 to 3 C₁-C₄ alkyl, and or 1-3 methoxy (OMe) or ethoxy (OEt) groups,
X is C₁-C₄ alkanediyl,
or a stereoisomer, a tautomer, a hydrate, a solvate, or a salt thereof, or a mixture of same.

6. A lipid nanoparticle (LNP) preparation comprising a compound of any one of claims 1-4.

7. An LNP preparation comprising:
a. a compound of any one of claims 1-4;
b. a phospholipid;
c. a sterol;
d. a conjugate linker lipid

8. An LNP preparation according to claim 6 or 7, that further comprises a pharmaceutical or therapeutical composition.

9. An LNP preparation according to any of claims 6 to 8, which further comprises an RNA.

10. A method for administering a therapeutic and/or prophylactic agent to a subject in need thereof, the method comprising administering the LNP preparation of any claims 6-9 to the subject.

11. A method for treating a disease or a disorder in a subject in need thereof, the method comprising administering the LNP preparation of any one of claims 6-9 to the subject, wherein the therapeutic and/or prophylactic agent is effective to treat the disease.

12. A method of producing a polypeptide of interest in a mammalian cell, the method comprising contacting the cell with the LNP preparation of any one of claims 6-9, wherein the therapeutic and/or prophylactic agent is or comprises an mRNA, and wherein the mRNA encodes the polypeptide of interest, whereby the mRNA is capable of being translated in the cell to produce the polypeptide of interest.

13. A method of specifically delivering a therapeutic and/or prophylactic agent to a mammalian organ, tissue, cell, or population of cells, the method comprising contacting a mammalian organ, tissue, cell, or population of cells, with the LNP preparation of any one of claims 6-9 whereby the therapeutic and/or prophylactic agent is delivered to the organ, tissue, cell, or population of cells.

14. A method of vaccinating by administering the LNP preparation of any one of claims 6-9.
